# EUROPEAN PATENT SPECIFICATION

(11) **EP 3 801 507 B1**
(45) Date of publication and mention of the grant of the patent: **01.01.2025**
(21) Application number: 19728338.5
(22) Date of filing: 23.05.2019
(51) Int. Cl.: A61K 31/421, A61K 31/4418, A61K 31/122, A61K 31/4439, A61K 31/454, A61K 36/062, A61K 36/07, A61K 36/09, A61K 36/74, A61P 25/00, A61P 25/28, A61P 31/20, A61P 9/10

(54) **TOMM6-INTERACTING EXTRACTS AND COMPOUNDS FOR USE IN THE TREATMENT AND PROPHYLAXIS OF NERVOUS SYSTEM DISEASES, ATHEROSCLEROSIS, HEPATITIS B INFECTION AND HUMAN PAPILLOMA VIRUS (HPV) INFECTION**
TOMM6-INTERAGIERENDE EXTRAKTE UND VERBINDUNGEN ZUR VERWENDUNG IN DER BEHANDLUNG UND PROPHYLAXE VON NERVENSYSTEMERKRANKUNGEN, ATHEROSKLEROSE, HEPATITIS-B-INFEKTION UND INFEKTION MIT HUMANEM PAPILLOMVIRUS (HPV)
EXTRAITS ET COMPOSÉS INTERAGISSANT AVEC TOMM6 DESTINÉS À ÊTRE UTILISÉS DANS LE TRAITEMENT ET LA PROPHYLAXIE DE MALADIES DU SYSTÈME NERVEUX, DE L'ATHÉROSCLÉROSE, D'UNE INFECTION PAR LE VIRUS DE L'HÉPATITE B ET D'UNE INFECTION PAR LE PAPILLOMAVIRUS HUMAIN (PVH)

(30) Priority: 24.05.2018 EP 18174145
(43) Date of publication of application: 14.04.2021
(60) Divisional application: 21190816.5
(73) Proprietor: ETH Zurich, 8092 Zurich (CH)
(72) Inventor: QUITTERER, Ursula, 8057 Zurich (CH); ABDALLA, Said, 55122 Mainz (DE)
(74) Representative: Kasche & Partner
(86) International application number: PCT/EP2019/063318
(87) International publication number: WO 2019/224300

(56) References cited:
- CN-A- 105 596 321
- FR-A1- 2 476 072
- KR-B1- 101 841 604
- JUI-CHIH CHANG: "Regulatory role of mitochondria in oxidative stress and atherosclerosis", WORLD JOURNAL OF CARDIOLOGY, vol. 2, no. 6, 1 January 2010 (2010-01-01), pages 150, XP055467851, ISSN: 1949-8462, DOI: 10.4330/wjc.v2.i6.150
- MANSOURI ABDELLAH ET AL: "Mitochondrial Dysfunction and Signaling in Chronic Liver Diseases", GASTROENTEROLOGY, ELSEVIER INC, US, vol. 155, no. 3, 2 August 2018 (2018-08-02), pages 629 - 647, XP085461158, ISSN: 0016-5085, DOI: 10.1053/J.GASTRO.2018.06.083
- RAJ KENNETH ET AL: "E1 [and] E4 Protein of Human Papillomavirus Type 16 Associates with Mitochondria", JOURNAL OF VIROLOGY, vol. 78, no. 13, 1 July 2004 (2004-07-01), US, pages 7199 - 7207, XP093178177, ISSN: 0022-538X, DOI: 10.1128/JVI.78.13.7199-7207.2004
- FISICARO PAOLA ET AL: "Targeting mitochondrial dysfunction can restore antiviral activity of exhausted HBV-specific CD8 T cells in chronic hepatitis B", NATURE MEDICINE, vol. 23, no. 3, 6 February 2017 (2017-02-06), New York, pages 327 - 336, XP093178169, ISSN: 1078-8956, Retrieved from the Internet <URL:http://www.nature.com/articles/nm.4275> DOI: 10.1038/nm.4275
- HOFFMANN ET AL: "HIGHER RATE OF NEUROPSYCHIATRIC ADVERSE EVENTS LEADING OT DOLUTEGRAVIR DISCONTINUATION IN WOMEN AND OLDER PATIENTS", HIV MEDICINE,, vol. 18, no. 18, 1 January 2017 (2017-01-01), pages 56 - 63, XP002789818
- PFANNER ET AL: "MITOCHONDRIAL BIOGENESIS: THE TOM AND TIM MACHINE", CURRENT BIOLOGY,, vol. 7, 1 January 1997 (1997-01-01), pages R100 - R103, XP002789819, DOI: 10.1016/S0960-9822(06)00048-0
- DATABASE BIOSIS [online] BIOSCIENCES INFORMATION SERVICE, PHILADELPHIA, PA, US; 2008, KORIYAMA Y ET AL: "A retinol binding protein, purpurin protects injury-induced apoptosis and regenerate axons in rat CNS neurons", XP002795707, Database accession no. PREV201200148561
- SOCIETY FOR NEUROSCIENCE ABSTRACT VIEWER AND ITINERARY PLANNER, vol. 38, 2008, 38TH ANNUAL MEETING OF THE SOCIETY-FOR-NEUROSCIENCE; WASHINGTON, DC, USA; NOVEMBER 15 -19, 2008
- MORI ET AL.: "TOXICITY AND TUMORIGENICITY OF PURPURIN, A NATURAL HYDROXYANTHRAQUINONE IN RATS: INDUCTION OF BLADDER NEOPLASMS", CANCER LETTERS, vol. 102, 1996, pages 193 - 198, XP002795708
- DI BARTOLO ET AL.: "TRAIL-DEFICIENCY ACCELERATES VASCULAR CALCIFICATION IN ATHEROSCLEROSIS VIA MODULATION OF RANKL", PLOS ONE, vol. 8, no. 9, E74211, 2013, pages 1 - 9, XP002795709
- KACZANOWSKA ET AL.: "DESING, SYNTHESIS AND IN VITRO EVALUATION OF NOVEL AMINOMETHYL-PYRIDINES AS DPP-4 INHIBITORS", ACS MEDICINAL CHEMISTRY LETTERS, vol. 1, 2010, pages 530 - 535, XP002795710

## Description

The present invention is directed to a TOMM6 (Translocase of Outer Membrane 6kDa subunit homologue; Mitochondrial import receptor subunit TOM6 homolog)-interacting compound of formula II for use in the treatment or prophylaxis of a nervous system disease, atherosclerosis, Hepatitis B infection and/or human papilloma virus (HPV) infection.

Nervous system diseases or disorders, also known as nervous system or neurological diseases or disorders, refer to medical conditions affecting the nervous system. This category encompasses central nervous and peripheral nervous diseases or disorders, including genetic disorders, seizure disorders (such as epilepsy), conditions of cardiovascular origin (such as stroke), congenital and developmental disorders (such as spina bifida), degenerative disorders such as multiple sclerosis (MS), Parkinson's disease (PD), and amyotrophic lateral sclerosis (ALS) and forms of dementia such as vascular dementia, Lewy Body dementia, frontotemporal dementia, and Alzheimer's disease (AD). Currently, there is a wide range of treatments for central and peripheral nervous system diseases and disorders. These can range from surgery to neural rehabilitation or prescribed medications.

AD is the most frequent form of dementia and on the rise worldwide. Major neuropathological features related to AD and other nervous system diseases are characterized by protein aggregation, neurotoxic tau filaments and mitochondrial dysfunction.

Age is the best-established non-genetic risk factor for dementia and AD, and with increasing life expectancy, the incidence of dementia is on the rise world-wide. Currently, there is no cure for dementia, including AD. There are only four different drugs approved for the treatment of AD: three different acetylcholinesterase inhibitors, which enhance the availability of the cognition-enhancing acetylcholine, and the NMDA receptor antagonist memantine (Kulshreshtha and Piplani, Neurol. Sci. 37, 1403-1435 (2016)). All of these drugs have major side effects. Also, the currently used drugs can only retard the disease progression by several months and cannot modify disease progression and/or relief AD symptoms only for a short time period. Therefore, there is an urgent need for disease-modifying treatment approaches for AD. A possible target is the aberrant protein aggregation process leading finally to Abeta plaque formation. However, approaches that only interfere with Abeta plaque formation have not demonstrated efficacy in retarding AD progression, and even showed major side effects (Kulshreshtha and Piplani, Neurol. Sci. 37, 1403-1435 (2016)). The underlying reason could be the fact that the sole increase in Abeta aggregates does not necessarily cause substantial neuronal loss (Calderon-Garciduenas & Duyckaerts, Handb. Clin. Neurol. 145, 325-337, 2017).

TOMM6 (Translocase of Outer Membrane 6kDa subunit homolog; Mitochondrial import receptor subunit TOM6 homolog) is an essential component of the TOMM machinery, i.e. the multisubunit translocases in the outer mitochondrial membrane, which are required for the import of nucleus-encoded precursor proteins. The TOMM machinery contains import receptors for the binding of cytosolically synthesized preproteins and the general import pore (GIP). Tomm20, Tomm22 and Tomm70 are the import receptors for preproteins. These receptors are attached to the other components of the Tom machinery, which form the core complex. Tomm6 is a subunit of the core complex, which together with the other subunits (Tomm40, Tomm22, Tomm7, Tomm5) is embedded in the outer membrane and forms the translocation pore (Dembowski et al., J. Biol. Chem. 276, 17679-17685, 2001). Tomm6 stabilizes the interaction between the receptors, specifically Tomm22, and the general insertion pore (Dekker et al., Mol. Cell. Biol. 18, 6515-6525, 1998). However, the *in vivo* role of TOMM6 was not investigated so far. Moreover, a pathophysiological role of TOMM6 has not been established.

A pathophysiological relevance between the Tomm machinery and late-onset Alzheimer's was proposed for TOMM40 with the S/VL and VL/VL (VL: very long) genotype of TOMM40 being reported to result in a higher expression of TOMM40 and apparently in a better protection against the APOE e3/3 background (Zeitlow et al., Biochim. Biophys. Acta 1863, 2973-2986, 2017). However, several studies did not replicate this observation, and the possible association between the TOMM40 genotype and AD is still under discussion (Roses et al., Alzheimers Dement 12, 687-694, 2016). The complexity of gene polymorphism-disease association could be the reason for difficulties to reproduce the initial data. These problems are also reflected by the fact that translation of genetic data into identification of novel targets for drug treatment of AD usually has not occurred (Lutz et al., Curr. Neurol. Neurosci. Rep 16, 48 (2016)). Thus, there have been no reports of successful treatment approaches for dementia or AD that involve TOMM40 and/or the TOMM machinery. In view of ongoing discussions about the relationship between TOMM40 genotypes and late-onset AD, researchers ask for more basic science data (Roses et al., Alzheimers Dement 12, 687-694, 2016). In this respect, Tomm40 knockout mice could help to elucidate the function of Tomm40 *in vivo.* Knockout of Tomm40 in mice is lethal. Unpublished data are available from heterozygous Tomm40 knockout mice, which have a reduced performance, a 30 % higher mortality than wild-type mice and motor defects (unpublished data by R. Zeh were cited by Zeitlow et al., Biochim. Biophys. Acta 1863, 2973-2986, 2017). However, aged heterozygous Tomm40 knockout mice did not develop defects of behaviour, learning, memory and/or other typical symptoms of AD (R. Zeh., unpublished). These observations further demonstrate that to date there is no established causality between TOMM40 or any other member of the TOMM machinery with neurodegeneration and/or AD. Kaczanowska et al. disclose the synthesis and evaluation of aminomethyl-pyridines as DPP-4 inhibitors (ACS Med. Chem. Lett. 2010,1,530-535).

Anthraquinones (also called anthracenediones) and specifically 9,10-anthraquinones are used as digester additive in the production of paper pulp by alkaline processes, like the Kraft-, the alkaline sulfite- or the Soda-AQ-processes. Anthraquinones also have utility as drugs, in particular as laxatives (e.g. dantron, emodin, aloe emodin, and some of the senna glycosides), antimalarials (e.g. rufigallol), antineoplastics (treatment of cancer, mitoxantrone, pixantrone, and the anthracyclines), and DNA dyes / nuclear counterstains (e.g. DRAQ5, DRAQ7 and CyTRAK Orange for use in flow cytometry and fluorescence microscopy). However, anthraquinones such as, e.g. rhein, emodin, aloe emodin, physcion, and chrysophanol can also be toxic, e.g. by causing hepatomyoencephalopathy in children.

Some natural pigments are anthraquinones and derivatives of anthraquinone, which are found, e.g., in aloe latex, senna, rhubarb, cascara buckthorn, fungi, lichens, and some insects. Generally, anthraquinone-comprising plant families include but are not limited to *Rubiaceae, Verbenaceae, Bignoniaceae, Rhamnaceae, Polygonaceae, Leguminosae, Scropulariaceae, Fabaceae,* and *Liliaceae.* The *Rubiaceae* are a family of flowering plants, commonly known as the coffee, madder, or bedstraw family. This family is mostly known as a source of a variety of alkaloids, the most familiar of which is quinine, one of the first agents effective in treating malaria. Woodruff (*Galium odoratum*) is a small herbaceous perennial that contains coumarin, a natural precursor of warfarin, and the South American plant *Carapichea ipecacuanha* is the source of the emetic ipecac. The leaves of the Kratom plant (*Mitragyna speciosa*) contain a variety of alkaloids, including several psychoactive alkaloids and are traditionally prepared and consumed in Southeast Asia for both painkilling and stimulant qualities. It functions as a p-opioid receptor agonist, and is often used in traditional Thai medicine similar to opioids, and often as a replacement for opioid painkillers like morphine.

The *Galium* and *Rubia* genera of the *Rubiaceae* family are known to comprise glycosides of colored anthraquinone derivatives and related hydroxyanthraquinone compounds (Hill and Richter, J. Chem. Soc. 1714-1719, 1936). There are several studies, which show antioxidant effects of *Rubia cordifolia* L. root extracts, which contain a wide variety of non-anthraquinone antioxidants, e.g. mollugin, terpenes, glycosides etc. (Priya and Siril, Int. J. Pharm. Sci. Rev. Res. 25, 154-164, 2014). Antioxidant effects are comparable to vitamin E and C (Priya and Siril, Int. J. Pharm. Sci. Rev. Res. 25, 154-164, 2014). However, a panoply of data from controlled clinical trials shows that the sole treatment with antioxidants has no significant effect in patients with nervous system diseases such as, e.g., dementia, AD and cardiovascular diseases (Farina et al., Cochrane Database Syst. Rev. 4:CD002854, 2017; Gugliandolo et al., Int. J. Mol. Sci. 18, E2594, 2017; Reddy AP & Reddy PH, Prog. Mol. Biol. Transl. Sci. 146, 173-201, 2017; Baradaran et al., J. Res. Med. Sci. 19, 358-367, 2014).

Chitra and Kumar (Int. J. Pharm Tech Res. 1, 1000-1009, 2009) investigated the effect of *Rubia cordifolia L.* root extract in mice, which received intracerebroventricular injection of a synthetic peptide, Abeta25-35, to cause neuronal damage. It is true that the synthetic peptide Abeta25-35 causes neurotoxicity and neuronal cell death. But for the following reasons, the acute brain damage induced in this mouse model has nothing to do with the human Alzheimer's disease in patients. (1) Chitra and Kumar used a synthetic peptide, the Abeta25-35 peptide. (2) This synthetic Abeta25-35 peptide does **not** occur in human brain under physiological conditions (Kubo et al., J. Neurosci. Res. 70, 474-483 (2002)). (3). Moreover, this synthetic Abeta25-35 peptide does **not** occur in brains of AD patients (Kubo et al., J. Neurosci. Res. 70, 474-483, 2002). (4) In contrast to the synthetic Abeta25-35 peptide used by Chitra and Kumar, only the protease-resistant [D-Ser26]-Abeta25-35/40 fragment accumulates over years in low quantities in brains of AD patients (Kubo et al., J. Neurosci. Res. 70, 474-483, 2002). (5) This naturally occurring beta-amyloid [D-Ser26]-Abeta25-35/40 fragment, which is racemized at Serine26 residue to yield D-Serine26, is highly resistant to degradation by proteinases and therefore can accumulate during the pathogenesis of AD, which takes decades to develop (Kaneko et al., Neuroscience 104, 1003-1011, 2001). (6) Because Chitra and Kumar tested the acute neurotoxicity of a single injection of a synthetic Abeta25-35 peptide, which is not detectable in healthy or diseased human brain, neither without nor with Alzheimer's disease, this mouse model is irrelevant for the pathogenesis of AD in humans, which requires decades to develop. For the same reasons, data obtained with this model are also irrelevant for the treatment of patients with AD. Hence, this study has no relevance for the human Alzheimer disease and AD patients.

It is the objective of the present invention to provide new means for use in the treatment and/or prophylaxis of a nervous system disease or disorder, preferably a human nervous system disease or disorder, preferably a central nervous system (CNS) or peripheral nervous system (PNS) disease or disorder, atherosclerosis, hepatitis B infection and HPV infection.
In a first aspect, the present invention is directed to a TOMM6 (Translocase of Outer Membrane 6kDa subunit homologue)-interacting compound according to the claims for use in the treatment or prophylaxis of a disease selected from the group consisting of: (a) nervous system disease or disorder, preferably a human nervous system disease or disorder, preferably a central nervous system (CNS) or peripheral nervous system (PNS) disease or disorder; (b) atherosclerosis; (c) Hepatitis B infection and (d) human papilloma virus (HPV) infection.

It was surprisingly found that interaction, e.g. binding, induction, stabilization and/or activation, of TOMM6 with a compound for use in the present invention is sufficient to (A) prevent mitochondrial dysfunction, toxic protein aggregation, Abeta plaque formation, PHF-tau formation and neuronal loss, (B) to treat and/or prevent atherosclerosis, atherosclerosis-induced neurodegeneration, diabetic neuropathy and autonomic neuropathy (for which atherosclerosis are major risk factors; Meyer et al., Diabet. Med. 21, 746-751, 2004) and other atherosclerosis-induced diseases, e.g. stroke, vascular dementia, coronary artery disease, myocardial infarction, (C) to inhibit the aggregation and assembly of HBsAg, thus having utility in the treatment of Hepatitis B infection, and (D) to treat infections with human papillomaviruses (HPV), preferably by inhibition of amyloidogenic protein aggregation. Without wishing to be bound by theory, human HPV infections rely on aggregation of the highly amyloidogenic protein E1^E4, also known as the HPV E4 protein (McIntosh et al., 82, 8196-8203 (2008). Notably, HPV E4 can constitute between 20 % and 30 % of the total wart protein (Doorbar et al., EMBO J. 5, 355-362 (1986)) and its expression relies on self-aggregation (Bryan et al., Virology 241, 49-60 (1998)).

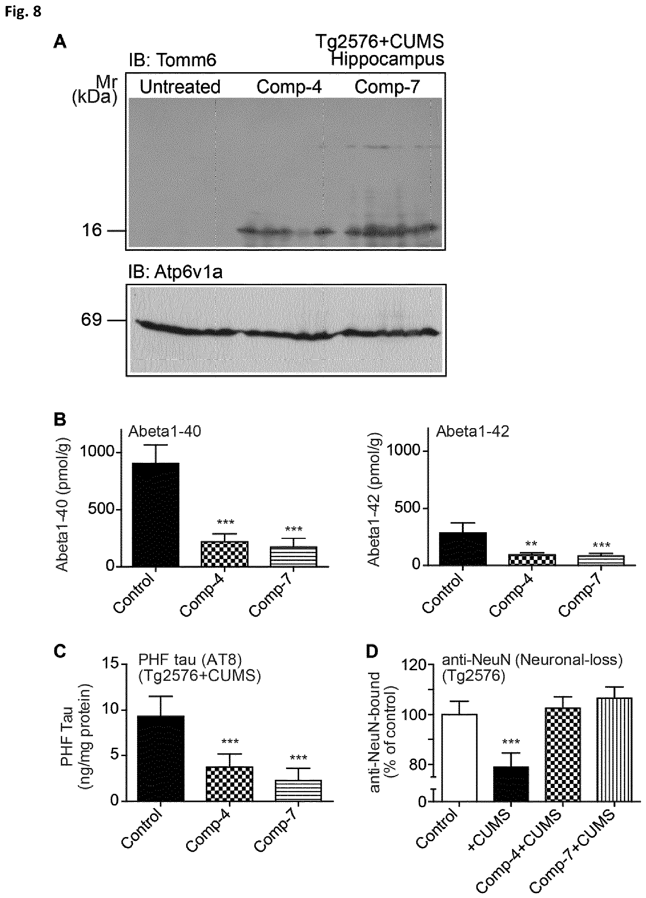

Last but not least, compounds of formula (II) were identified as TOMM6 -interacting compounds (Examples 13 and 14 below) which induce TOMM6 and, thus, decrease hippocampal PHF tau hyperphosphorylation (Figure 8C) and retard overt neuronal loss in AD model mice, i.e. retard major symptoms of AD and neurodegeneration *in vivo* (Figure 8D).

In the context of the present invention, the term "TOMM6" refers to the Translocase of Outer Membrane 6kDa subunit homolog and the Mitochondrial import receptor subunit TOMM6 homolog, which are synonyms. Preferably, TOMM6 is a protein comprising, preferably consisting of an amino acid sequence selected from the group of SEQ ID NO: 1, 2 and 3. The term "TOMM6" also preferably encompasses homologs of TOMM6, termed "TOMM6 homolog(s)", with TOMM6 activity that comprise, preferably consist of an amino acid sequence having a sequence identity of at least 85%, preferably 90%, more preferably 95%, most preferably 98% sequence identity with the amino acid sequence of TOMM6, preferably with an amino acid sequence selected from the group consisting of SEQ ID NO: 1, 2 and 3.

The percentage identity of related amino acid molecules can be determined with the assistance of known methods. In general, special computer programs are employed that use algorithms adapted to accommodate the specific needs of this task. Preferred methods for determining identity begin with the generation of the largest degree of identity among the sequences to be compared. Preferred computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

In the context of the present invention, the term "TOMM6-interacting compound or composition" is meant to encompass any compound or composition that (i) directly or indirectly binds to TOMM6 and thus stabilizes and/or enhances its physiological activity, preferably vs. an untreated control (as can be determined, e.g., in the assay of Example 9 below), and/or (ii) induces the expression (increases the protein content) of TOMM6 in a cell, preferably vs. an untreated control (as can be determined, e.g. in an immunoblot assay using anti-TOMM6 antibodies, e.g. as described in Example 13 below). The terms "induction, stabilization and/or activation of TOMM6", as used herein, also refer to any stabilization and/or enhancement of TOMM6's physiological activity and/or induction of expression of TOMM6 as defined above.

It is noted that the compounds selected from the group consisting of Formula (II) are TOMM6-inducing compounds, which are generally devoid of any antioxidant activity. Hence, an antioxidant activity of TOMM6-interacting compounds is not a prerequisite for the technical effect of the compounds and extracts for use in the present invention. Moreover, the compounds of Formulae (II) without antioxidant activity also show neuroprotective activity and prevent neuronal loss in AD models. Thus, cell-protective TOMM6-stabilizing activity of small molecule compounds does not require antioxidant activity.

In summary, the Examples herein demonstrate that TOMM6-interaction (e.g. induction, stabilization and/or activation) by compounds of Formula (II) (e.g. Compounds 3-10) are sufficient to prevent mitochondrial dysfunction, toxic protein aggregation, Abeta plaque formation, PHF-tau formation and neuronal loss.

The compounds described herein are generally named by using the nomenclature that was computed based on the structural drawings by the software ACD/Chemsketch 2015 provided by Advanced Chemistry Development, Inc., Canada and BIOVIA Draw 2016 provided by BIOVIA, USA. The compounds can be obtained by chemical synthesis and/or by extraction from plants or other organisms, preferably fungal organisms.

For compounds having asymmetric centers, it is understood that, unless otherwise specified, all of the optical isomers and mixtures thereof are encompassed. Each stereogenic carbon may be in the (*R)*- or (*S)*- configuration or a combination of configurations if not indicated differently. Also, compounds with two or more asymmetric elements can be present as mixtures of diastereomers. Furthermore, the compounds of the present invention preferably have a diastereomeric purity of at least 50%, preferably at least 60%, 70%, 80%, 85%, more preferably at least 90%, 95%, 96%, 97%, most preferably at least 98%, 99% or 100%. In addition, compounds with carbon-carbon double bonds may occur in *Z-* and *E-* forms, with all isomeric forms of the compounds being included in the present invention unless otherwise specified. Where a compound exists in various tautomeric forms, a recited compound is not limited to any one specific tautomer, but rather is intended to encompass all tautomeric forms.

Recited compounds are further intended to encompass compounds in which one or more atoms are replaced with an isotope, *i.e.,* an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, preferably at least 90%, 95%, or 98%. Some embodiments of the compounds have an enantiomeric excess of at least 99%. It will be apparent that single enantiomers (optically active forms) can be obtained by asymmetric synthesis, synthesis from optically pure precursors, biosynthesis, extraction from plants or organisms, or by resolution of the racemates, e.g. enzymatic resolution or resolution by conventional methods such as crystallization in the presence of a resolving agent, or chromatography, using, for example, a chiral HPLC column.

As used herein, a "substituent" or "residue" or **"R",** refers to a molecular moiety that is covalently bound to an atom within a molecule of interest. For example, a "substituent", **"R"** or "residue" may be a moiety such as a halogen, alkyl group, haloalkyl group or any other substituent described herein that is covalently bonded to an atom, preferably a carbon or nitrogen atom, that forms part of a molecule of interest. The term "substituted" as used herein, means that any one or more hydrogens on the designated atom is replaced with a selection from the indicated substituents, provided that the designated atom's normal valence is not exceeded, and that the substitution results in a stable compound, *i.e.,* a compound that can be isolated and characterized using conventional means. For example, substitution can be in the form of an oxygen bound to any other chemical atom than carbon, e.g. hydroxyl group, or an oxygen anion. When a substituent is oxo, *i.e.,* =O, then 2 hydrogens on the atom are replaced. An oxo group that is a substituent of an aromatic carbon atom results in a conversion of -CH- to -C(=O)- and a loss of aromaticity. For example, a pyridyl group substituted by oxo is a pyridone.

The term "heteroatom" as used herein shall be understood to mean atoms other than carbon and hydrogen such as and preferably O, N, S and P.

If a first compound, a substituent or a residue ends, e.g., in the name "-yl", this ending indicates that the first compound, substituent or residue is covalently bound to a second compound, substituent or residue.

It is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. "(C₁₋₁₀)alkyl" denotes a hydrocarbon residue containing from 1 to 10 carbon atoms, e.g. a methyl, ethyl, propyl, *iso*-propyl, *n*-butyl, *iso*-butyl, *sec*-butyl, *tert*-butyl, *n*-pentyl, *iso*-pentyl, *n*-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), iso-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, preferably 1, 2, 3, 4, 5, or 6 double bond(s).

The expression "alkynyl" refers to at least partially unsaturated, substituted or non-substituted straight-chain or branched hydrocarbon groups that contain the number of carbon items indicated, e.g. "(C₂₋₁₀)alkynyl" denotes a hydrocarbon residue containing from 2 to 10 carbon atoms, for example an ethinyl, propinyl, butinyl, acetylenyl, or propargyl group. Preferably, alkynyl groups have one or two (especially preferably one) triple bond(s).

Furthermore, the terms "alkyl", "alkenyl" and "alkynyl" refer to groups in which one or more hydrogen atom(s) have been replaced, e.g. by a halogen atom, preferably F or Cl, such as, for example, a 2,2,2-trichloroethyl or a trifluoromethyl group.

The term "carbocycle" shall be understood to mean a substituted or non-substituted hydrocarbon cycle containing the number of carbon items indicated, e.g. "(C₃₋₁₀)carbocycle" or from 3 to 20, preferably from 3 to 12 carbon atoms, more preferably 5 or 6 carbon atoms. These carbocycles may be either aromatic or non-aromatic systems. The non-aromatic ring systems may be mono- or polyunsaturated.

The term "carbobicycle" refers to a carbocycle as defined above comprising more than 1 ring, preferably two rings. Preferred carbocycles and carbobicycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl, benzocycloheptenyl, spiro[4,5]decanyl, norbornyl, decalinyl, bicyclo[4.3.0]nonyl, tetraline, or cyclopentylcyclohexyl. The carbo- and/or carbobicyclic residue may be bound to the remaining structure of the complete molecule by any atom of the cycle, which results in a stable structure

The term "carbocycle" shall also include "cycloalkyl" which is to be understood to mean aliphatic hydrocarbon-containing rings preferably having from 3 to 12 carbon atoms. These non-aromatic ring systems may be mono- or polyunsaturated, i.e. the term encompasses cycloalkenyl and cycloalkynyl.

The term "heterocycle" refers to a stable substituted or non-substituted, aromatic or non-aromatic, preferably 3 to 20 membered, more preferably 3-12 membered, most preferably 5 or 6 membered, monocyclic, heteroatom-containing cycle. Each heterocycle consists of carbon atoms and one or more, preferably 1 to 4, more preferably 1 to 3 heteroatoms preferably chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

The term "heterobicycle" refers to a heterocycle as defined above comprising more than 1 ring, preferably two rings.

The hetero- and/or heterobicyclic residue may be bound to the remaining structure of the complete molecule by any atom of the cycle, which results in a stable structure. Exemplary heterocycles and heterobicycles include, but are not limited to pyrrolidinyl, pyrrolinyl, morpholinyl, thiomorpholinyl, thiomorpholinyl sulfoxide, thiomorpholinyl sulfone, dioxalanyl, piperidinyl, piperazinyl, tetrahydrofuranyl, 1-oxo-λ4-thiomorpholinyl, 13-oxa-11-aza-tricyclo[7.3.1.0-2,7]tridecy-2,4,6-triene, tetrahydropyranyl, 2-oxo-2H-pyranyl, tetrahydrofuranyl, 1,3-dioxolanone, 1,3-dioxanone, 1,4-dioxanyl, 8-oxa-3-aza-bicyclo[3.2.1]octanyl, 2-oxa-5-aza-bicyclo[2.2.1]heptanyl, 2-thia-5-aza-bicyclo[2.2.1]heptanyl, piperidinonyl, tetrahydro-pyrimidonyl, pentamethylene sulphide, pentamethylene sulfoxide, pentamethylene sulfone, tetramethylene sulphide, tetramethylene sulfoxide and tetramethylene sulfone, indazolyl, benzimidazolyl, benzodioxolyl, imidazolyl, 1,3-benzodioxolyl and pyrazolyl.

The expressions "alkyl/alkenyl/alkynyl ether" refer to a saturated or non-saturated, straight-chain or branched hydrocarbon group that contains the number of carbon items indicated. For example, "(C₁₋₁₀)alkyl ether" denotes a hydrocarbon residue containing from 1 to 10 carbon atoms, and any suitable number of oxygen atoms that will result in an ether structure. Alkyl/alkenyl/alkynyl ether groups as used herein shall be understood to mean any linear or branched, substituted or non-substituted alkyl/alkenyl/alkynyl chain comprising an oxygen atom either as an ether motif, i.e. an oxygen bound by two carbons. The ether residue can be attached to the Formulas provided in the present invention either via the carbon atom or via the oxygen atom of the ether residue.

The "substituent" or "residue" or **"R"** as used herein, preferably **R^{1a}**, **R^{1b}**, **R^{2a}**, **R^{2b}**, **R^{3a}**, **R^{3b}**, **R^{4a}**, **R^{4b}**, **R⁵, R⁶**, **R⁷, R⁸**, **R^{9a}**, **R^{9b}**, and/or **R¹⁰** can be attached directly to the Formulae provided in the present invention or by means of a linker. Said linker may be a polyethyleneglycol. The term polyethyleneglycol as used herein refers to a chain of substituted or non-substituted ethylene oxide monomers. If the forumulae and description note the residues **R¹**, **R²**, **R³**, **R⁴**, and/or **R⁹** (i.e. without the letters "a" or "b"), then these residues can be residues as defined for either **R^{1a}/R^{1b}**, **R^{2a}/R^{2b}**, **R^{3a}/R^{3b}**, **R^{4a}/R^{4b}**, and **R^{9a}/R^{9b}**.

As used herein, the terms "nitrogen" or "N" and "sulphur" or "S" include any oxidized form of nitrogen and sulphur and the quaternized form of any basic nitrogen as long as the resulting compound is chemically stable. For example, for an -S-C₁₋₆ alkyl radical shall be understood to include -S(O)-C₁₋₆alkyl and -S(O)₂-C₁₋₆alkyl.

As used herein, a wording defining the limits of a range of length such as, e. g., "from 1 to 5" or "(C₁₋₅)" means any integer from 1 to 5, i. e. 1, 2, 3, 4 and 5. In other words, any range defined by two integers explicitly mentioned is meant to comprise and disclose any integer defining said limits and any integer comprised in said range.

By way of example, the term "substituted in *meta* position or substituted in *para* position", as used herein, means that a compound is either substituted by at least one given substituent in *para* position to the position where the compound is attached to another compound or residue, or substituted in two of its *meta* positions by at least one substituent. As denoted above for the *para* position, the *meta* position denotes the position *meta* to the position where the compound is attached to another compound or residue.

The invention includes pharmaceutically acceptable salts or solvates of the compounds of the present invention.

Pharmaceutically acceptable salts include those derived from pharmaceutically acceptable inorganic and organic acids and bases. Examples of suitable acids include hydrochloric, hydrobromic, sulphuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric and benzenesulfonic acids. Other acids, such as oxalic acid, while not themselves pharmaceutically acceptable, may be employed in the preparation of salts useful as intermediates in obtaining the compounds and their pharmaceutically acceptable acid addition salts. Salts derived from appropriate bases include alkali metal (e.g., sodium), alkaline earth metal (e.g. magnesium), ammonium and N-(C₁-C₄ alkyl)₄⁺ salts.

The compound of Formula(II) for use in the present invention is one according to claim 1 or 2.

In a preferred embodiment, the compound of Formula (II) for use in the present invention is selected from the group consisting of

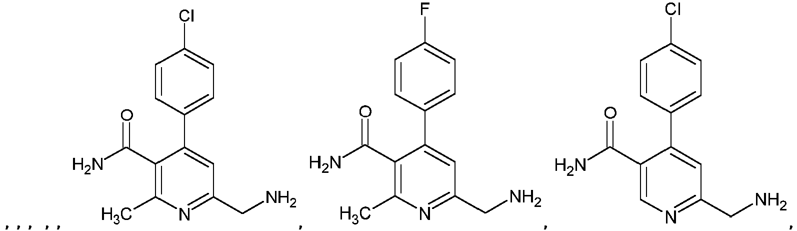

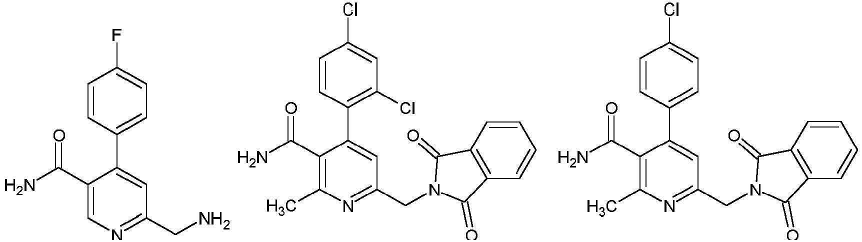

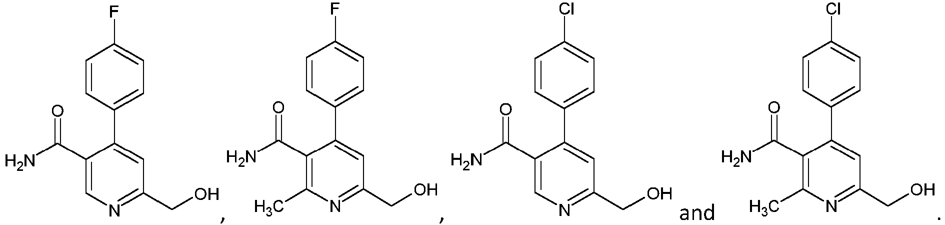

In a preferred embodiment, the TOMM6 (Translocase of Outer Membrane 6kDa subunit homologue)-interacting compound for use in the present invention is one, wherein the nervous system disease or disorder is selected from the group consisting of:
brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; subcortical leukoencephalopathy and subcortical atherosclerotic encephalopathy; mixed forms of dementia; M. Parkinson; progressive supranuclear palsy and other forms of atypical parkinsonism; frontotemporal dementia; subcortical dementia; CADASIL (cerebral autosomal dominant arteriopathy with subcortical infarcts and leukoencephalopathy); cerebral palsy;
encephalitis lethargica; corticobasal degeneration; multiple system atrophy; chronic traumatic encephalopathy; Lytico-Bodig disease; FTDP-17; Parkinson linked to chromosome-17; diabetic neuropathy; symptoms of depression and depression-related symptoms, preferably anhedonia and anorexia; schizophrenia with dementia; Korsakoff's psychosis; Lewy Body diseases; progressive supranuclear palsy; corticobasal degeneration; Pick's disease; Huntington's disease; thalamic degeneration; Creutzfeld-Jacob disease; HIV Dementia; disorders with mitochondrial dysfunction, preferably neurodegenerative diseases of ageing; cognitive-related disorder; mild cognitive impairment; age-associated memory impairment; age-associated cognitive decline; vascular cognitive impairment; central and peripheral symptoms of atherosclerosis and ischemia;
atherosclerosis-related cardiovascular diseases; stroke; perivascular disease; renal dysfunction and renal failure; stress-related disorders; attention deficit disorders; attention deficit hyperactivity disorders; and memory disturbances in children.

For therapeutic use, the compounds may be administered in any conventional dosage form in any conventional manner. Routes of administration include, but are not limited to oral, (trans)dermal, topical, nasal, parenteral, intravenous, intramuscular and subcutaneous administration, preferably injections. The preferred modes of administration are oral, nasal, topical, intravenous or subcutaneous.

The present invention also teaches a pharmaceutical composition comprising a TOMM6 (Translocase of Outer Membrane 6kDa subunit homologue)-interacting compound or composition for use in the present invention.

The compounds, compositions or pharmaceutical compositions for use in the invention may be administered alone or in combination with adjuvants that enhance stability of the compounds, facilitate administration of pharmaceutical compositions containing them in certain embodiments, provide increased dissolution or dispersion, increase activity, provide adjunct therapy (e.g. an acetylcholinesterase inhibitor, memantine, a renin-angiotensin system-inhibiting agent such as an ACE inhibitor and/or an AT1 antagonist, and/or an L-type calcium channel inhibitor such as amlodipine), and the like, including other active ingredients. Advantageously such combination therapies utilize lower dosages of the conventional therapeutics, thus avoiding possible toxicity and adverse side effects incurred when those agents are used as monotherapies. The above-described compounds and compositions may be physically combined with conventional therapeutics or other adjuvants into a single pharmaceutical composition. Reference in this regard may be made to Cappola et al.: U.S. patent application no. 09/902,822, PCT/US 01/21860 und US provisional application no. 60/313,527. Advantageously, the compounds or compositions may then be administered together in a single dosage form. In some embodiments, the (pharmaceutical) compositions comprising such combinations of compounds contain at least about 5 %, but more preferably at least about 20 %, of a compound for use in the present invention (w/w). The optimum percentage (w/w) of a compound of the invention may vary and is within the purview of those skilled in the art. Alternatively, the compounds may be administered separately (either serially or in parallel). Separate dosing allows for greater flexibility in the dosing regime.

As mentioned above, dosage forms of the compounds described herein include pharmaceutically acceptable carriers and adjuvants known to those of ordinary skill in the art. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from 1-500 mg/dose for a 70 kg patient. Although one dose per day may be sufficient, up to 5 doses per day may be given. For oral doses, up to 2500 mg/day may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician.

The term "TOMM6 homolog", as used herein, describes homologs of TOMM6 with TOMM6 activity that comprise, preferably consist of an amino acid sequence having a sequence identity of at least 85%, preferably 90%, more preferably 95% most preferably 98% sequence identity with the amino acid sequence of, preferably human or rodent TOMM6, preferably sequence identity with the amino acid sequence selected from the group consisting of SEQ ID NOs: 1, 2 and 3.

The following Figures and Examples serve to illustrate the invention and are not intended to limit the scope of the invention as described in the appended claims.

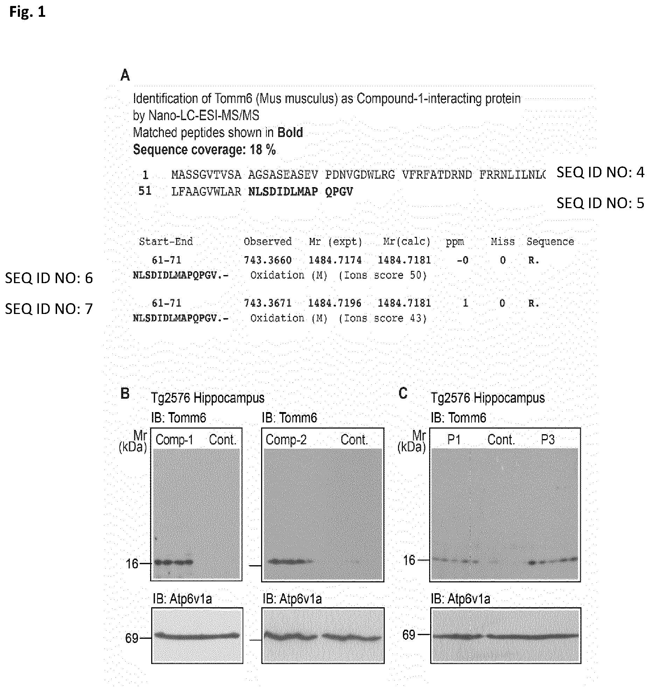

**Fig. 1** shows the identification of Tomm6 as an interacting/stabilizing protein of Compound-1, Compound-2, and extract P1 and P3 from *Galium aparine* and *Rubia cordifolia L.* (**A**) Solubilized hippocampal proteins from Tg2576 mice were incubated with Compound-1 at a concentration of 10 microM, proteins were concentrated by centrifugation over Amicon Ultra Centrifugal Filter Units (MWCO 3kDa), and separated by urea-containing SDS-PAGE under reducing conditions. Compound-1-interacting proteins were identified by co-migration, the protein band was cut and subjected to protein identification. Nano-LC-ESI-MS/MS analysis identified Tomm6 as a hippocampal protein, which interacts with Compound-1. **(B, C)** Immunoblot detection of hippocampal Tomm6 in aged 18-month-old Tg2576 mice without (Cont.) and with 6 months of treatment with Compound-1 (Comp-1) and Compound-2 (Comp-2) is shown in panels (**B**) and treatment with P1 extract from *Galium aparine* (P1) and P3 from *Rubia cordifolia L.* (P3) is shown in panel (**C**). The lower panels show control immunoblots detecting Atp6vla (V-type protein ATPase catalytic subunit A); (**B,** left panel n=4 mice/group; **B**, right panel n=5 mice/group; **C,** n=5 treated mice/group and n=4 controls).

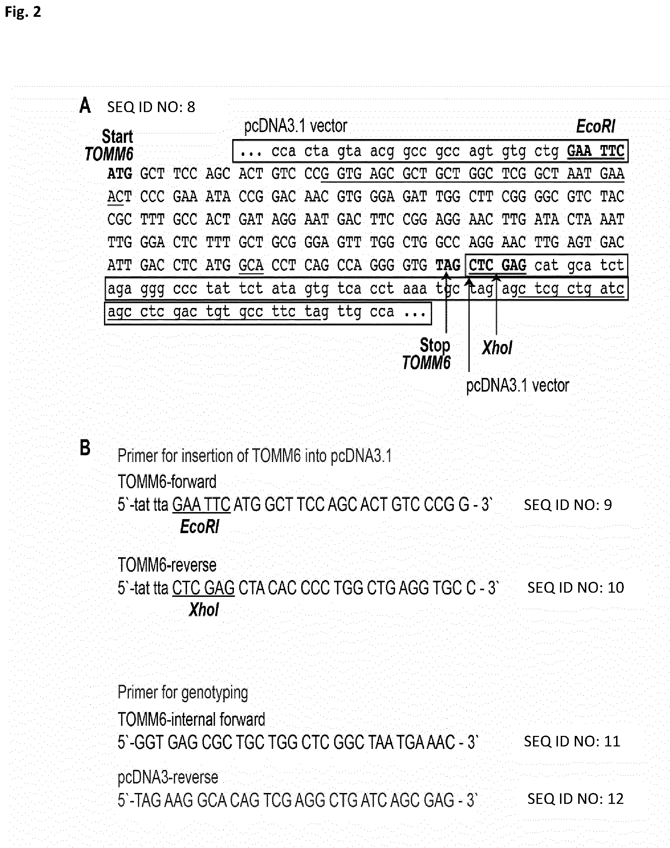

**Fig. 2** shows the construction of TOMM6 expression plasmid. (**A**) Insertion of TOMM6 cDNA into EcoRI and Xhol sites of pcDNA3.1 expression plasmid. (**B**) Sequences of PCR-primers used for construction of TOMM6-pcDNA3.1 plasmid, and for genotyping PCR are given.

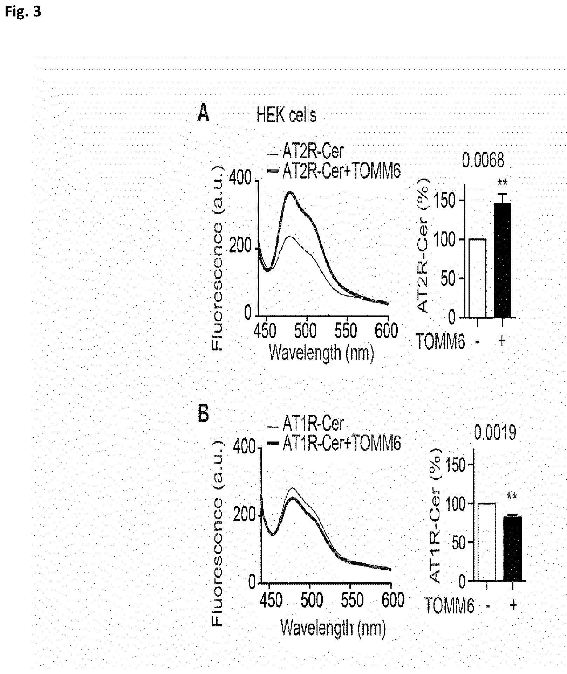

**Fig. 3** shows that TOMM6 enhances AT2R (AGTR2) protein folding in cells. (**A**) Exogenous co-expression of TOMM6 with AT2R-Cer in HEK cells significantly increased cell surface AT2R-Cer (AGTR2-Cerulean) protein levels as determined by fluorescence spectrophotometry of AT2R-Cer (±s.d., n=8). (**B**) Expression of TOMM6 with AT1R-Cer in HEK cells significantly decreased cell surface AT1R-Cer (AGTR1-Cerulean) protein levels (±s.d., n=8).

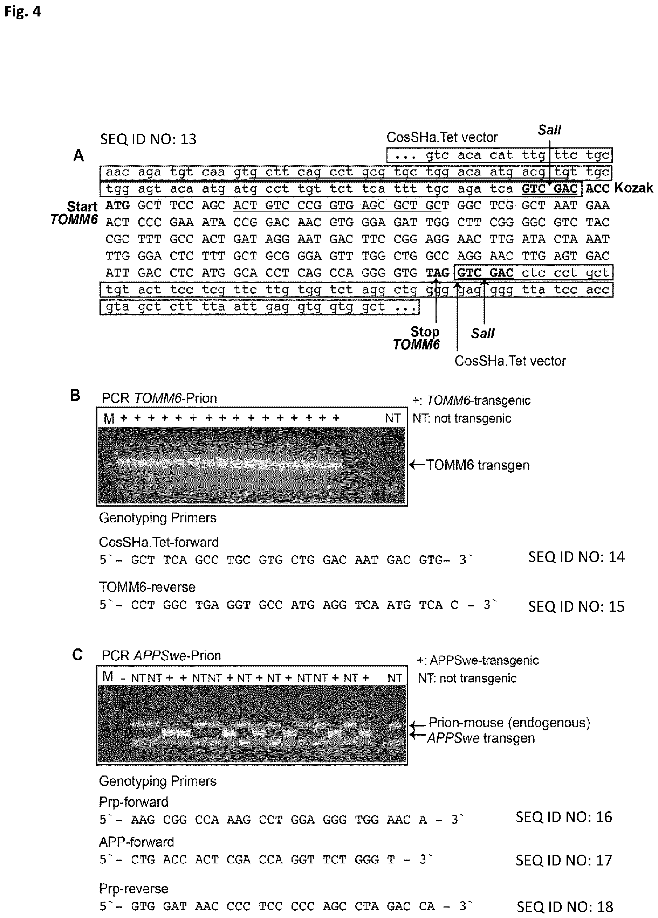

**Fig. 4** shows the generation of Tg-TOMM6 mice with neuron-specific expression of TOMM6. (**A**) Construction of the plasmid used for generation of Tg-TOMM6 mice. The cDNA encoding TOMM6 was inserted into the *Sall* site of the CosS.Ha.Tet vector. (**B, C**) Identification of double-transgenic Tg-2576-TOMM6 mice by genotyping PCR. Primer sequences for detection of TOMM6-transgen (**B**), and APPSwe-transgene are given (**C**). As an internal control, the endogenous *Prp* was co- amplified (**C**).

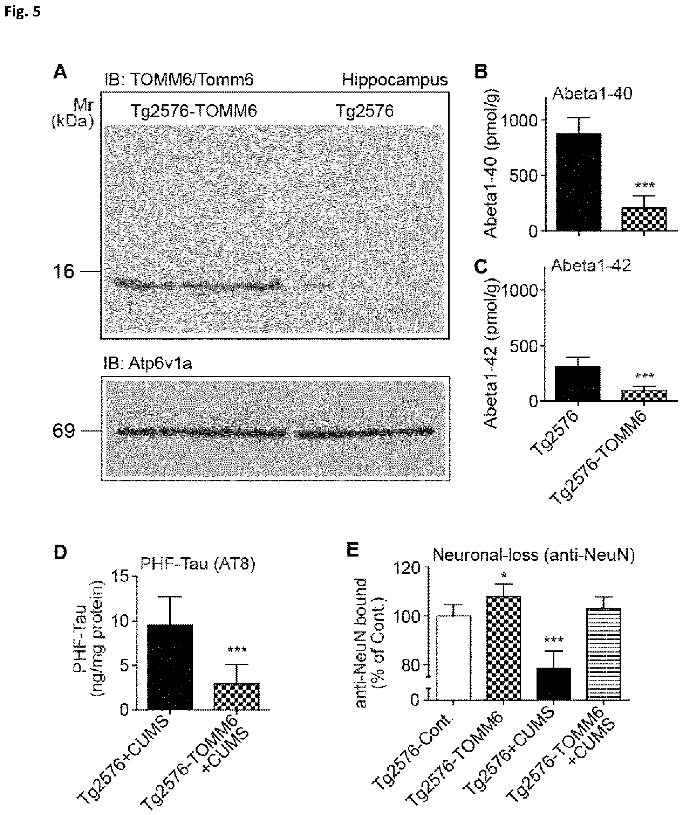

**Fig. 5** shows that transgenic TOMM6 expression retards major neuropathological features in the hippocampus of Tg2576 AD mice. (**A**): Immunoblot detection of hippocampal content of TOMM6/Tomm6 protein in 18 months-old double-transgenic Tg2576-TOMM6 mice (n=10) relative to single-transgenic Tg2576 controls (n=8). The lower panel shows a control immunoblot detecting mitochondrial Atp6vla (V-type protein ATPase catalytic subunit A). (**B, C**): Transgenic expression of *TOMM6* retarded the accumulation of SDS-insoluble Abeta1-40 (**B**) and Abeta1-42 (**C**) in the hippocampus of 18 months-old double-transgenic Tg2576-TOMM6 mice compared to single-transgenic Tg2576 control mice (±s.d., n=8 (Tg2576) and n=10 (Tg2576-TOMM6); ***, p<0.0001. (**D**): Transgenic *TOMM6* expression led to a decreased hippocampal content of hyperphosphorylated PHF-Tau (detected with AT8 antibody) in 15 months-old Tg2576-TOMM6 mice with 3 months of CUMS (±s.d., n=8; ***,p=0.0003). (**E**) Transgenic *TOMM6* expression prevented the hippocampal neuronal loss (detected with anti-NeuN antibody) in 15 months-old Tg2576-TOMM6 mice with 3 months of CUMS compared to single-transgenic Tg2576 mice subjected to CUMS (±s.d., n=8; ***, p<0.001 vs. Tg2576-Cont, Tg2576-TOMM6, and Tg2576-TOMM6+CUMS; *p<0.05 vs. Tg2576-Cont.; Tukey's test).

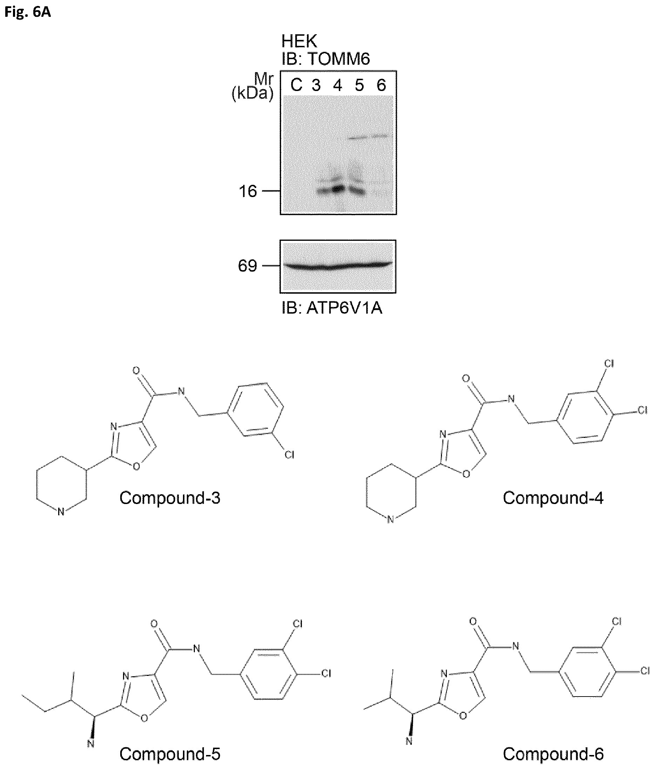

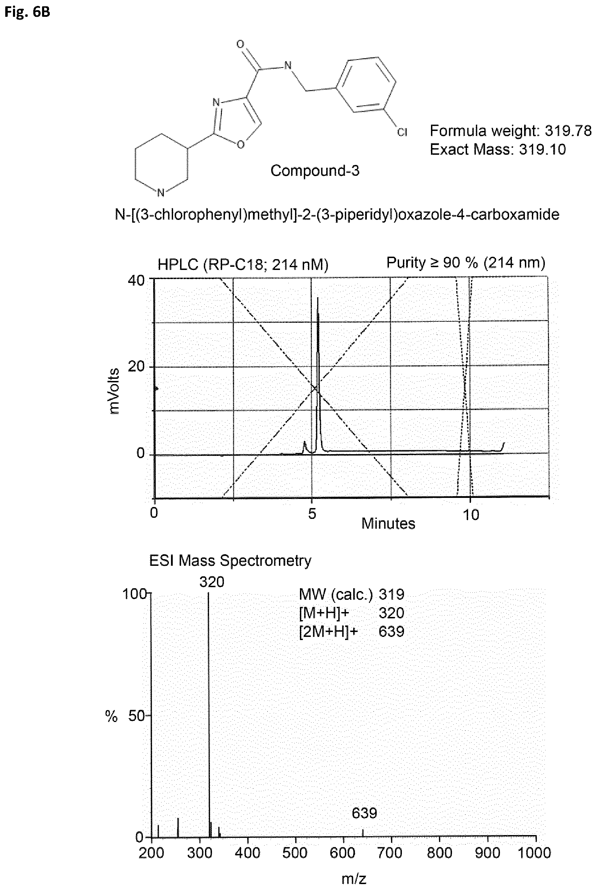

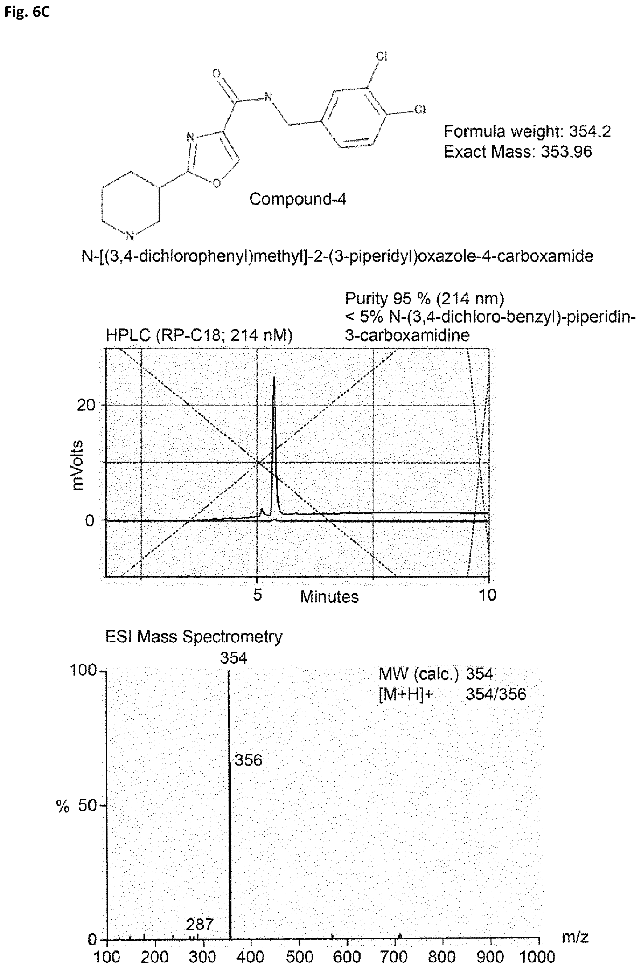

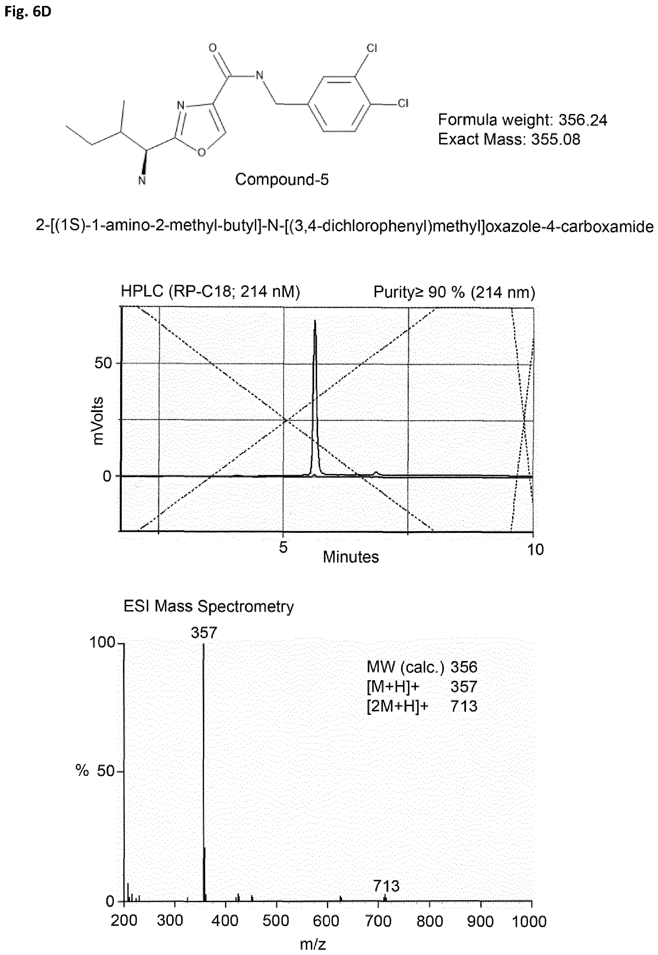

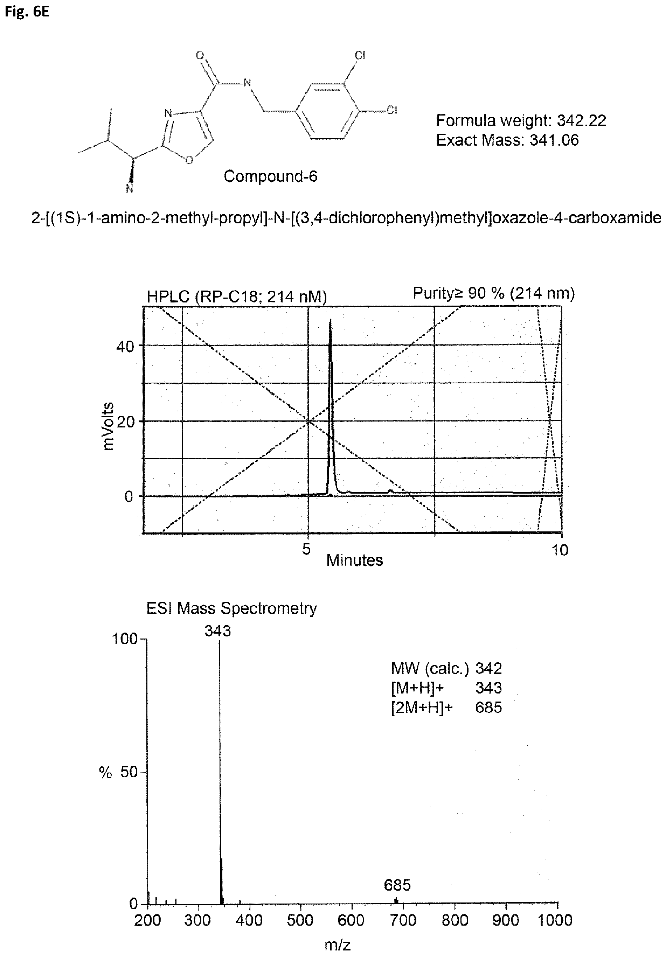

**Fig. 6** **shows that compounds 3-6, which are not antioxidants, increase neuroprotective TOMM6 in HEK cells.** (**A**): Immunoblot detection of TOMM6 protein in HEK cells cultured for 60 h without (control) or with compound-3, compound-4, compound-5, and compound-6 (final concentration 20 microM). The lower panel shows chemical formulas of compounds. (**B-E**): Analytical HPLC and ESI-MS data of compound-3 (**B**), compound-4 (**C**), compound-5 (**D**), and compound-6 (**E**).

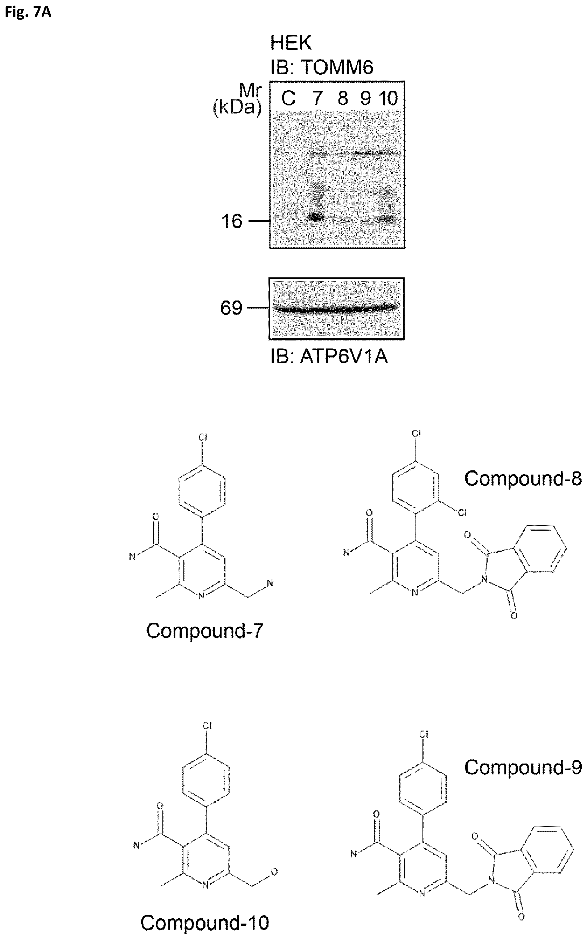

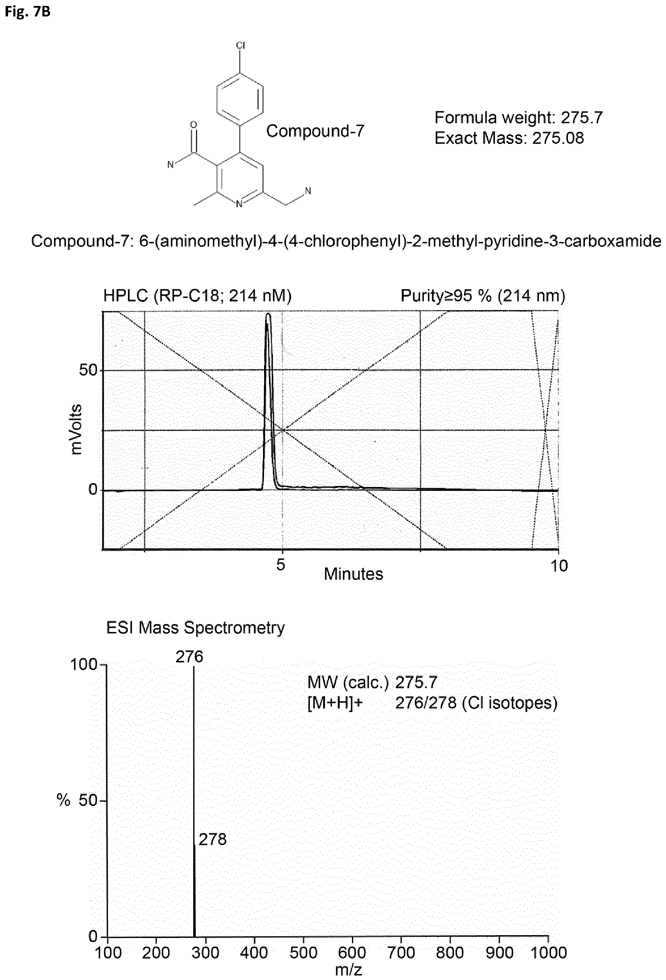

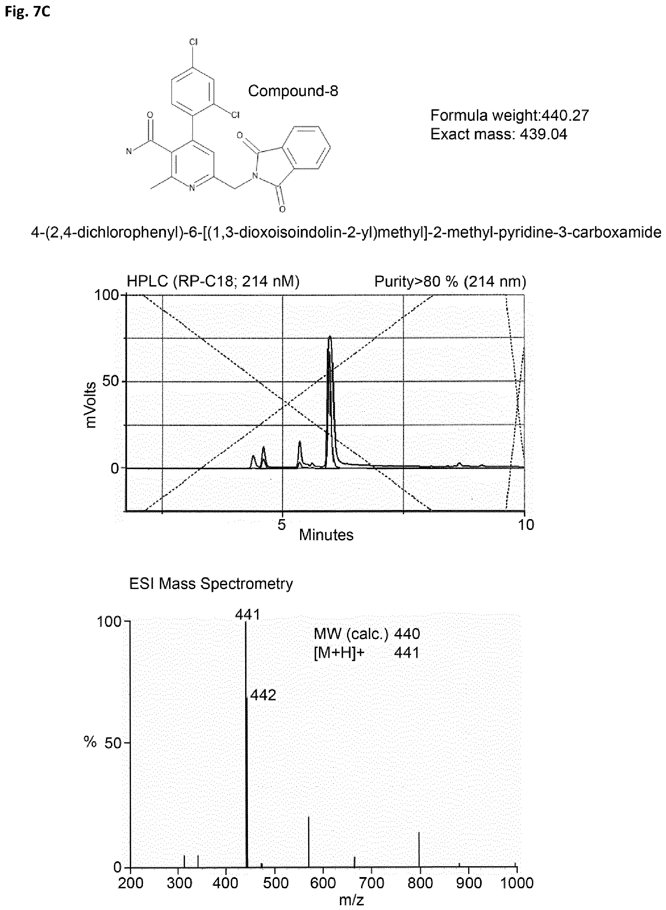

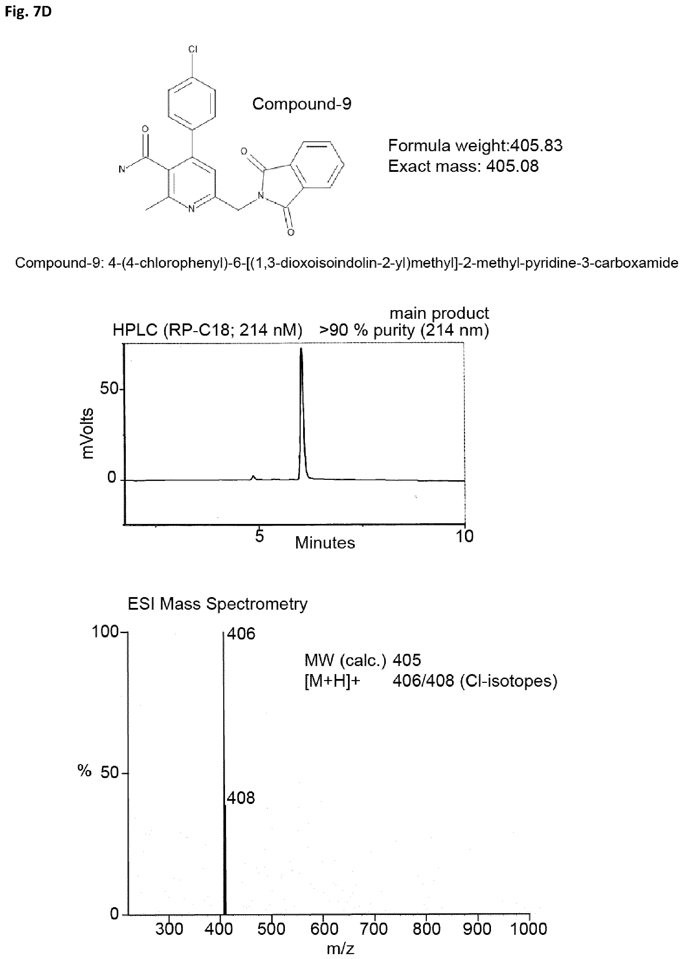

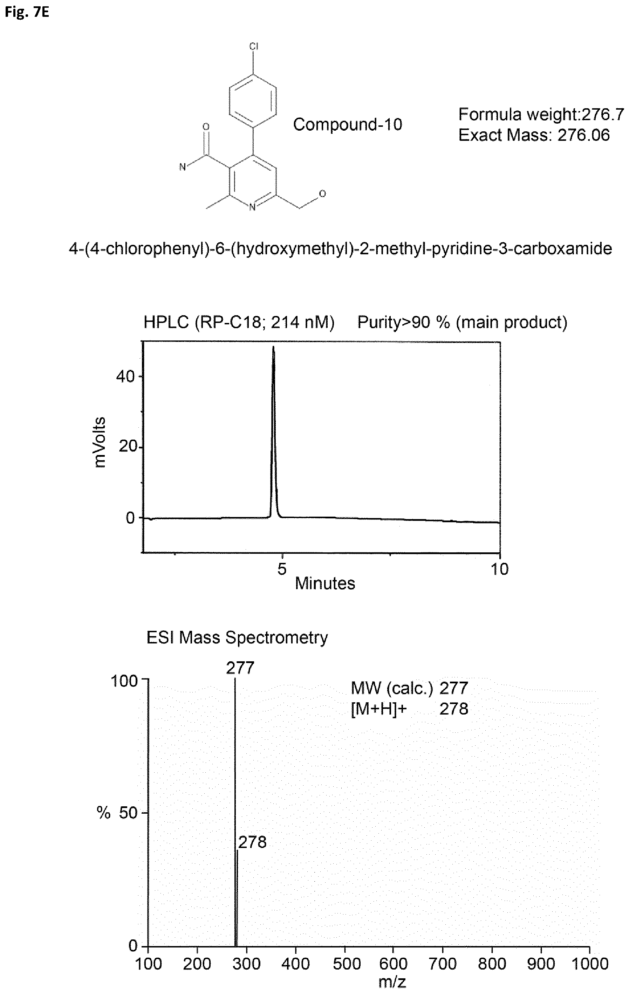

**Fig. 7** **shows that compounds 7-10, which are not antioxidants, increase neuroprotective TOMM6 in HEK cells. (A):** Immunoblot detection of TOMM6 protein in HEK cells cultured for 60 h without (control) or with compound-7, compound-8, compound-9, and compound-10 (final concentration 20 microM). The lower panel shows chemical formulas of compounds. (**B-E**): Analytical HPLC and ESI-MS data of compound-7 (**B**), compound-8 (**C**), compound-9 (**D**), and compound-10 (**E**).
**Fig. 8** **shows that TOMM6-interacting compound-4 and compound-7 inhibit symptoms of AD and neurodegeneration in AD model mice.** (**A**): Treatment with compound-4 and compound-7 of Tg2576 AD mice imposed to chronic mild stress by the CUMS protocol led to an increased hippocampal Tomm6 protein content (n=5-6 mice/group). (**B**): Treatment for 6 months with Tomm6-interacting compound-4 and compound-7 inhibited accumulation of aggregated hippocampal Abeta1-40 and Abeta1-42 in 18-month-old Tg2576 AD mice (±s.d., n=4, **, p<0.01; ***, p<0.001; Tukey's test). (**C,D**): Compound-4 and compound-7 inhibited hippocampal PHF tau hyperphosphorylation (**C**) and hippocampal neuronal loss (**D**) in 15 month-old Tg2576 AD mice subjected to the CUMS protocol (±s.d., n=8, ***,p<0.001; Tukey's test).

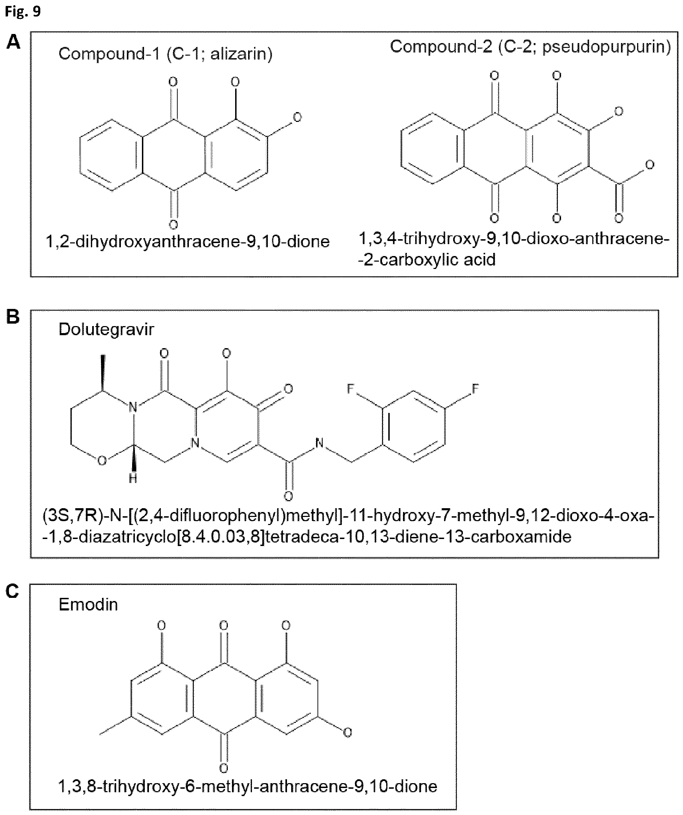

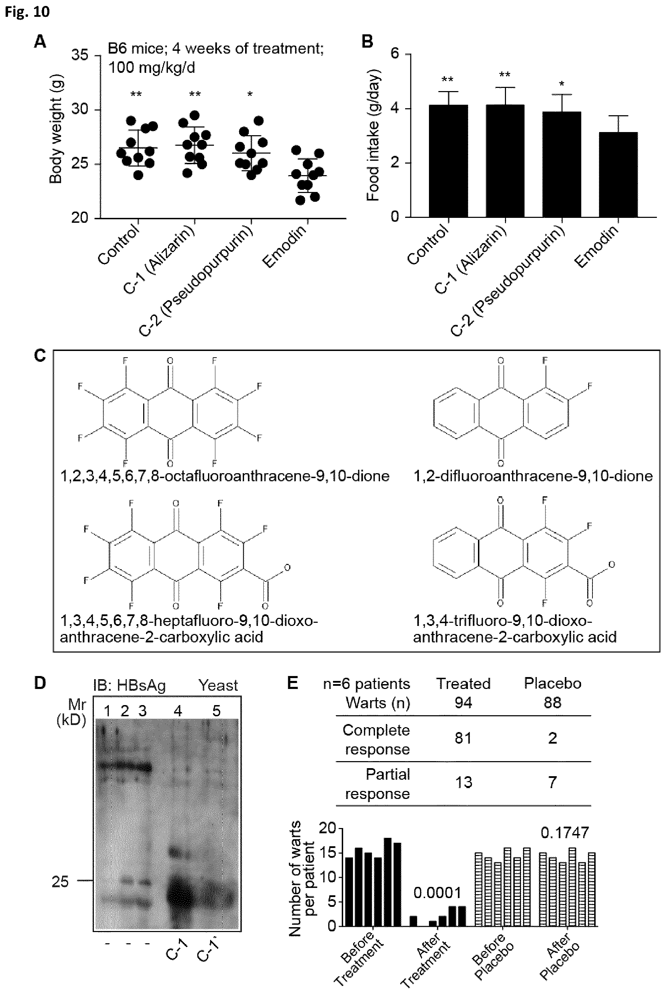

**Fig. 9** **shows formulas of alizarin (Compound-1) and pseudopurpurin (Compound-2) compared to dolutegravir and emodin. A,** Formula of Compound-1 and Compound-2. **B**, Formula of the tricyclic metal cation-binding dolutegravir, which is not a Tomm6 inducer (cf. Figure 10) and has neuropsychiatric side effects. **C,** Formula of emodin, which is toxic, causes diarrhea, and leads to decreased food intake and weight loss (cf. Figure 10).
**Fig. 10** **shows analysis of emodin in vivo. A,** Body weight of 12-week-old B6 mice after treatment for 4 weeks without (Control) and with alizarin (Compound-1), pseudopurpurin (Compound-2) or emodin at a once daily oral dose of 100 mg/kg/day. **B**, Daily food intake of B6 mice during four weeks (8-12 weeks of age) of treatment without and with alizarin (Compound-1), pseudopurpurin (Compound-2) or emodin at a once daily oral dose of 100 mg/kg/day. Data shown in **A** and **B are** means±s.d. (n=10; *,p<0.05 and **,p<0.01 vs. emodin, Tukey's test). **C,** Formulas indicating the positions of claimed halogen and fluorine-substitutions of anthrachinone compounds. **D,** Immunoblot detection of HBsAg protein in yeast (IB: SAG) shows that alizarin (C-1: 10 microg/ml; C-1': 1 microg/ml) prevents HBsAg aggregation, which is induced by overexpression in yeast. **E**, Treatment effect of topical Compound-1 treatment on wart lesion size of six voluntary research participants. Topical treatment was performed with Compound-1-containing hydrogel (0.2 %) and vehicle (placebo). The numbers of warts with complete response and partial response are given (upper panel). The lower panel shows the total number of warts before and after treatment with Compound-1 (left) and with placebo (right). Statistical significance is indicated (paired t-test).

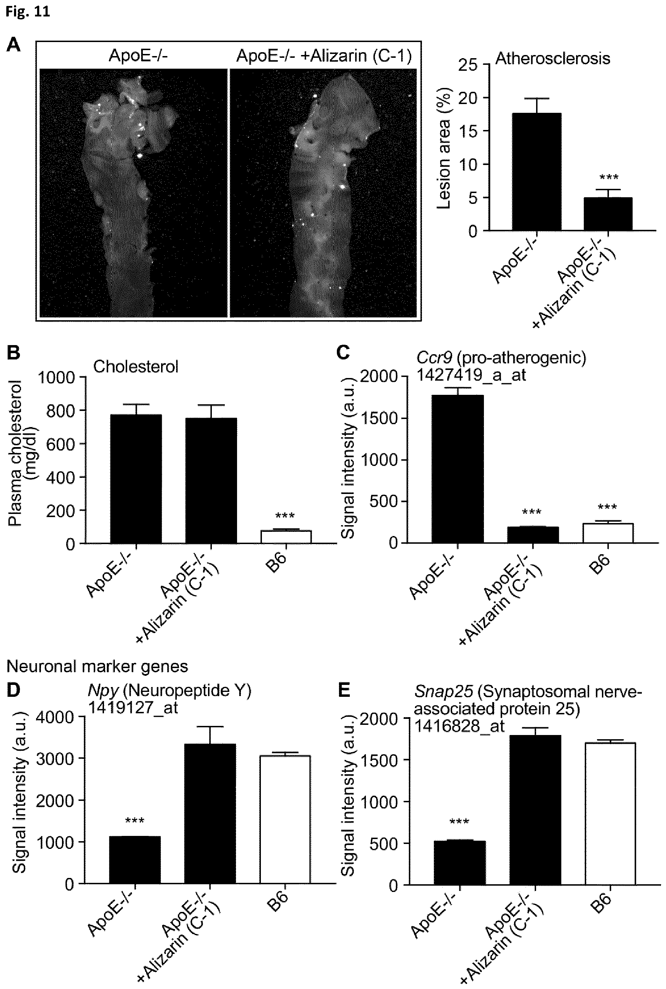

**Fig. 11****. Alizarin retards the development of atherosclerosis in ApoE-deficient mice as a model of atherosclerosis. A,** Atherosclerotic lesion area in the aorta of 9-month-old ApoE-deficient (ApoE-/-) mice treated without (ApoE-/-) and with alizarin (ApoE-/- + Alizarin (C-1) for 8 months. The left panel shows representative oil red O-stained aortas, and the right panel shows quantitative data (mean ± s.d.; n=4; ***,p<0.001, unpaired Student's t-test). **B,** Plasma cholesterol levels of untreated ApoE-/-, alizarin-treated ApoE-/-, and untreated non-transgenic B6 control mice (mean ± s.d.; n= 4; ***,p<0.001 vs. ApoE-/- and ApoE-/-+Alizarin, Tukey's test). **C,D,E,** Aortic gene expression data of pro-atherogenic *Ccr9* (**C**; probe set name: 1427419_a_at), and the neuronal marker genes *Npy* (**D**; probe set name: 419127_at) and *Snap25* (**E;** probe set name: 1416828_at). Aortic gene expression data are from two gene chips per group each performed with RNA from three aortas (mean ± s.d.; n=2; ***,p<0.01 vs. ApoE-/- (**C**), ***,p<0.01 vs. ApoE-/-+Alizarin and B6 (**D,E**), Tukey's test).

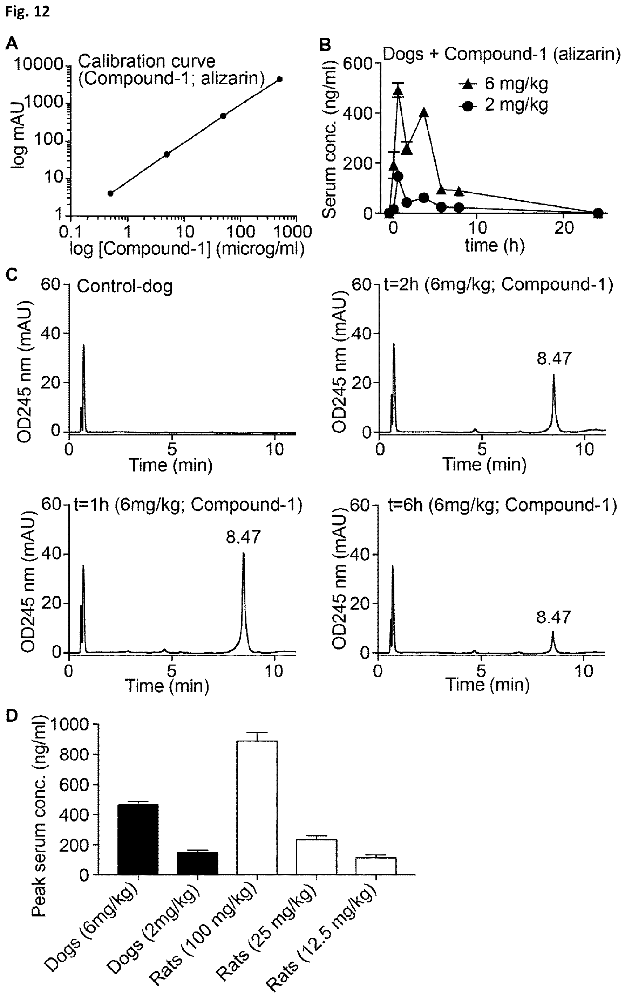

**Fig. 12** **shows the pharmacokinetic characterization of alizarin (Compound-1) in dogs and rats. A,** Calibration curve for quantitative determination of alizarin (Compound-1) concentration in serum by HPLC shows good linearity in the concentration range of 500 ng/ml - 500 microg/ml. **B,** Serum concentration of alizarin in dogs was determined by HPLC after repeated intake of alizarin for 4 weeks at a once daily oral dose of 2 mg/kg and 6 mg/kg. Serum was taken at the indicated time points after drug intake at t=0 (mean ± s.d.; n=3). **C,** Representative HPLC chromatograms of alizarin detection in serum taken from dogs without alizarin treatment (upper left panel) and with oral alizarin (Compound-1) treatment for 4 weeks at a once daily oral dose of 6mg/kg/d. Serum was taken at the indicated time points after oral intake of 6 mg/kg of alizarin. During the extraction of alizarin from serum, samples were concentrated 10-fold before HPLC analysis. **D,** Peak serum concentrations of alizarin (Compound-1) in dogs and rats were determined by HPLC after repeated once daily oral intake of the indicated dose of alizarin for 4 weeks. Serum was taken at t=1h after drug intake (mean ± s.d., n=3).

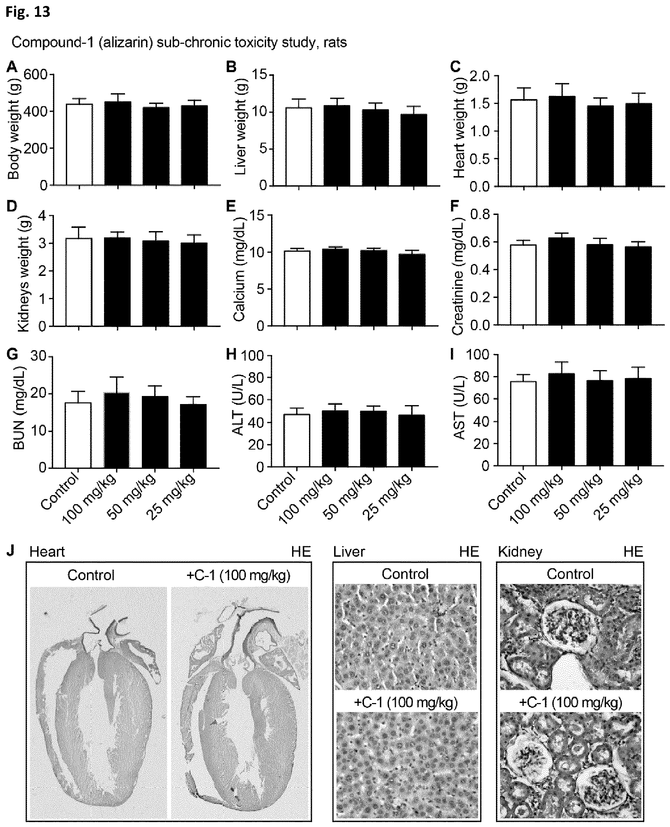

**Fig. 13** **shows a sub-chronic toxicity study of alizarin in rats, which documents a wide therapeutic index. A-I**, Sub-chronic toxicity of alizarin was analysed in rats by treatment for 3 months with a once daily oral dose of alizarin of 25 mg/kg/day, 50 mg/kg/day and 100 mg/kg/day starting at an age of 4 weeks. Alizarin at a once daily oral dose of 100 mg/kg, 50 mg/kg and 25 mg/kg had no significant adverse effect regarding body weight (**A**), liver weight (**B**), heart weight (**C**), kidney weight (**D**), and serum levels of calcium (**E**), creatinine (**F**), blood urea nitrogen (BUN; **G**), alanine transaminase (ALT; **H**) and aspartate aminotransferase (AST; **I**). Data are presented as mean ± s.d. (n=5). **J,** Histopathologic analysis of hematoxylin-eosin-stained paraffin sections of heart (left), liver (middle) and kidney (right) showed no adverse effects of alizarin in rats upon alizarin treatment for 3 months at a once daily oral dose of 100 mg/kg (+C-1, 100 mg/kg). Sections are representative of five animals per group.

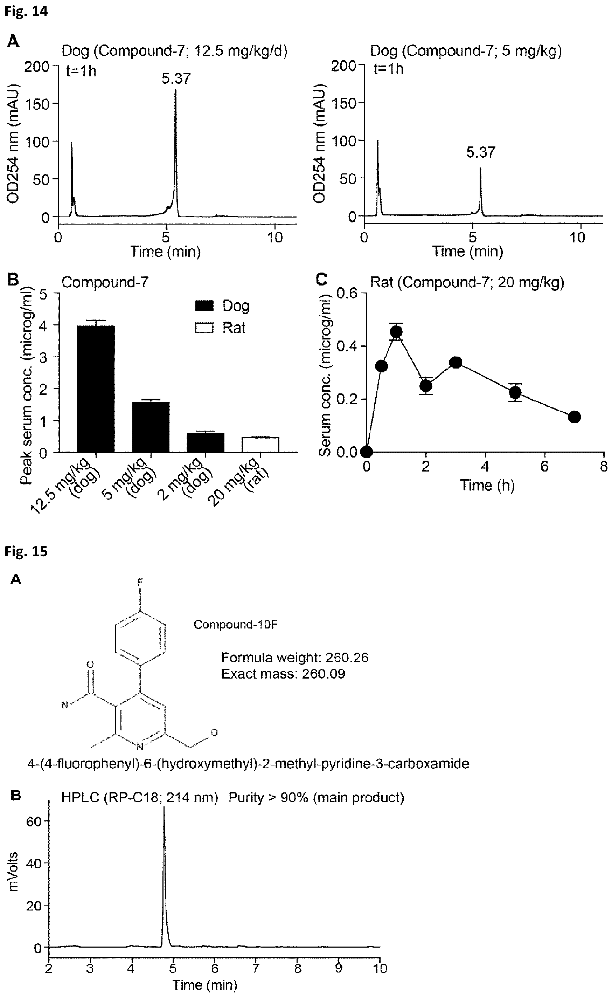

**Fig. 14** **shows pharmacokinetic data of Compound-7 in dogs and rats. A,** HPLC chromatograms of Compound-7 detection in serum of dogs after repeated once daily oral intake of 12.5 mg/kg and 5 mg/kg of Compound-7. Serum was taken at t=1h after drug intake. **B,** Peak serum concentrations of Compound-7 in dogs after repeated once daily oral intake of 12.5 mg/kg, 5 mg/kg and 2 mg/kg, and in rats dosed with 20 mg/kg/d (mean±s.d.; n=3). **C,** Serum concentration of Compound-7 in rats after repeated once daily oral intake of 20 mg/kg/d. Serum was taken from rats at the indicated time points after oral intake of 20 mg/kg of Compound-7 at t=0 (mean±s.d.; n=3).

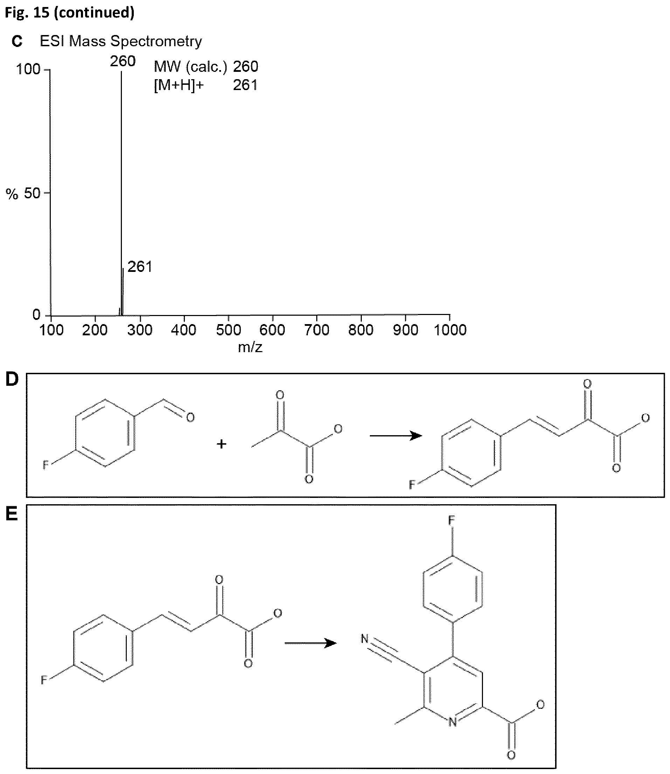

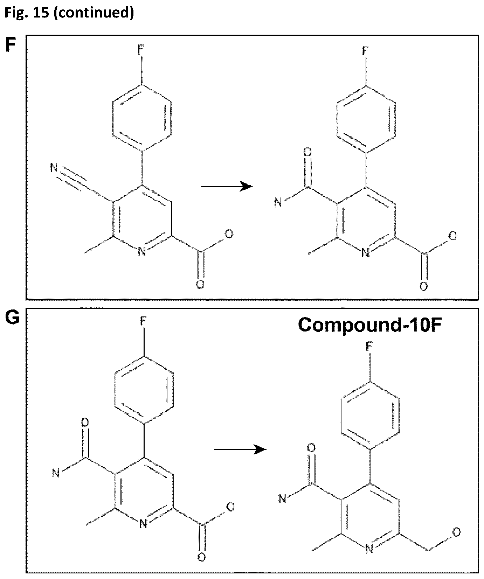

**Fig. 15** **shows the development of Compound-10F. A,** Formula of Compound-10F (4-(4-fluorophenyl)-6-(hydroxymethyl)-2-methyl-pyridine-3-carboxamide), which is the fluorine-substituted analogue of Compound-10. **B,** HPLC chromatogram of Compound-10F. **C,** ESI mass spectrometry analysis of Compound-10F. **D-G,** Chemical synthesis steps for Compound-10F. The analogous synthesis route was also used for synthesis of Compound-10 and Compound-7.

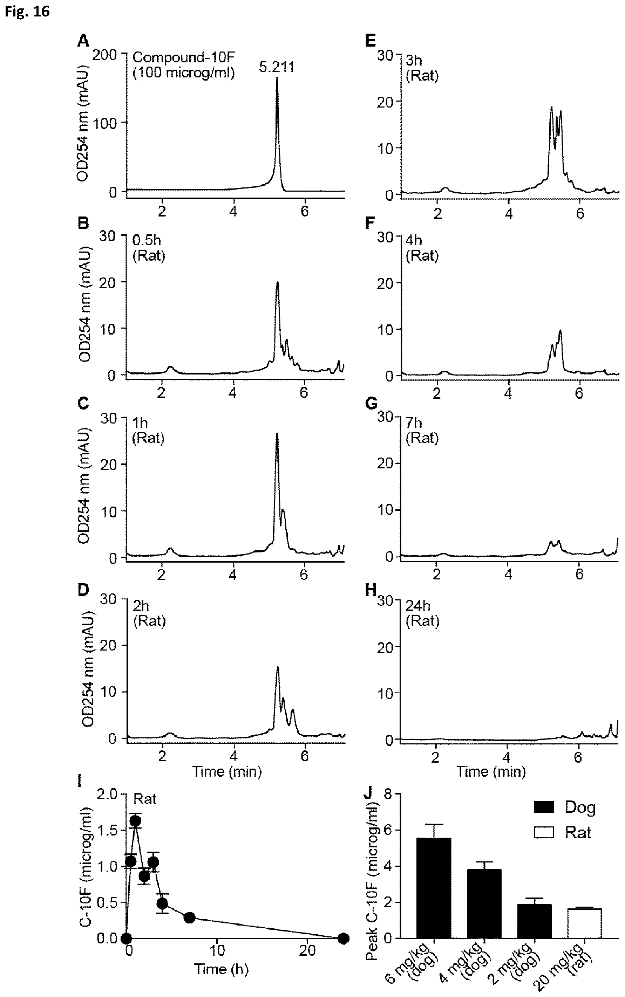

**Fig. 16** **shows pharmacokinetic data of Compound-10F. A,** HPLC chromatogram of Compound-10F spiked into rat serum at a concentration of 100 microg/ml. **B-H,** HPLC chromatograms of Compound-10F detection in rat serum after repeated oral intake of a once daily dose of 20 mg/kg. Rat serum was taken at the indicated time points after oral intake of Compound-10F. **I**, Serum concentration of Compound-10F in rats after repeated once daily oral intake of 20 mg/kg/d. Serum was taken from rats at the indicated time points after oral intake of 20 mg/kg of Compound-10F at t=0 (mean±s.d.; n=3). **J,** Peak serum concentrations of Compound-10F in dogs after repeated once daily oral intake of 6 mg/kg, 4 mg/kg and 2 mg/kg, and in rats dosed with 20 mg/kg/d (mean±s.d.; n=3).
**Fig. 173 shows that Compound-10F is a Tomm6 inducer and retards symptoms of AD and neurodegeneration in vivo. A,** Immunoblot detection of Tomm6 in hippocampal tissue of 15-month-old Tg2576 AD mice subjected to CUMS for three months and treated with Compound-10 and Compound-10F (10 mg/kg/d) during the CUMS protocol. The left panel shows immunoblot detection of hippocampal Tomm6, and the right panel shows quantitative data (mean±s.d., n=3; **,p<0.01 vs. untreated Tg2576+CUMS Control mice, Tukey's test). **B,** Hippocampal PHF-Tau content of Tg-2576 AD mice subjected to CUMS for three months and treated with Compound-10 and Compound-10F (10 mg/kg/d) during the CUMS protocol (mean ±s.d.; n=6; ***,p<0.001 vs. untreated Tg2576+CUMS mice, Tukey's test). **C,** CUMS-induced hippocampal neuronal loss was determined with anti-NeuN antibody of Tg2576 AD mice subjected to CUMS for three months and treated with Compound-10 and Compound-10F (10 mg/kg/d) during the CUMS protocol (mean ±s.d.; n=6; ***,p<0.001 vs. untreated Tg2576+CUMS mice, Tukey's test). Age-matched Tg2576 AD mice without CUMS served as controls (column 1).

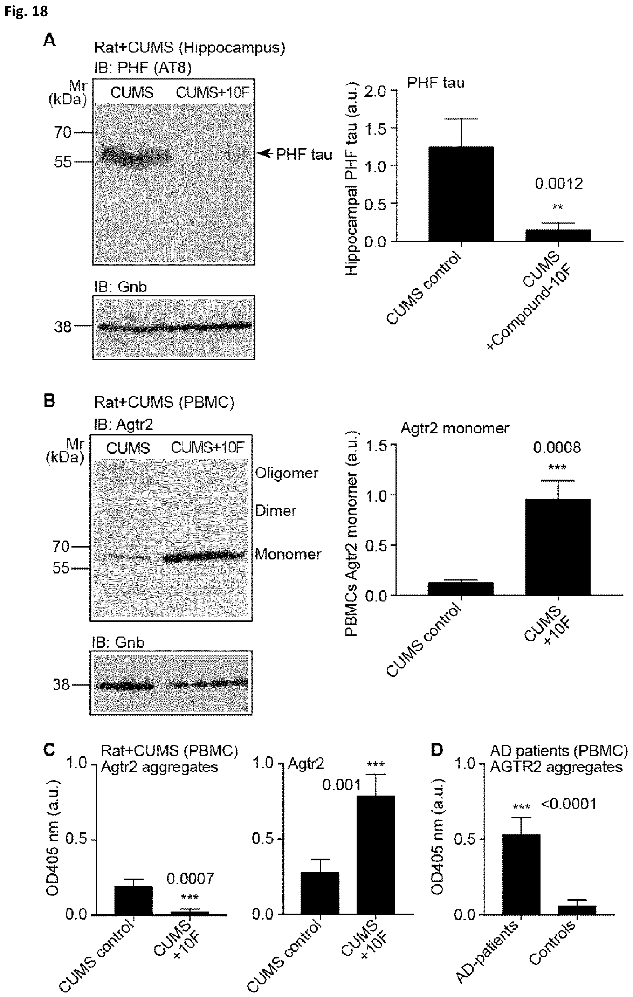

**Fig. 18** **shows that Compound-10F prevents hippocampal PHF tau hyperphosphorylation and peripheral Agtr2 (AT2R) aggregation in the CUMS model with early symptoms of sporadic AD. A,** Immunoblot detection of hippocampal hyperphosphorylated PHF tau in aged, 16-month-old rats with 4 weeks of CUMS without (CUMS control) and with treatment with Compound-10F during the CUMS protocol (CUMS+10F). The left panel shows immunoblot detection of hippocampal hyperphosphorylated PHF tau, and the right panel shows quantitative data (±s.d.; n=4; unpaired t-test). **B,** Immunoblot detection of Agtr2 (AT2R) in peripheral blood mononuclear cells (PBMCs) of aged 16-month-old rats with 4 weeks of CUMS and treatment without (CUMS) and with Compound-10F during the CUMS protocol (CUMS+10F). The left panel shows immunoblot detection of Agtr2 (AT2R) in PBMCs, and the right panel shows quantitative data of Agtr2 monomer contents (±s.d.; n=3 (CUMS); n=4 (CUMS+10F); unpaired t-test). **C,** Quantitative determination of aggregated Agtr2 protein and total Agtr2 protein by ELISA of PBMC of rats subjected to CUMS without (CUMS control) and with Compound-10F treatment during the CUMS protocol (CUMS+10F) (±s.d., n=4; p-values are indicated and were determined with the unpaired t-test). **D,** Quantitative determination of aggregated AGTR2 protein content by ELISA in PBMC of human Alzheimer Disease (AD) patients and age-matched healthy controls without dementia (±s.d.; n=6; the p-value was determined with the unpaired t-test).

### Example 1: Materials and Methods

### Animal models and generation of Tg-TOMM6 mice

(a) Tg2576 mice (Taconic Biosciences, Rensselaer, NY, USA) with overexpression of human APP695 with the double mutation K670N/M671L were used as a genetic model of familial AD (FAD) (see Hsiao et al., Science 274, 99-103 (1996)).
(b) Tg-TauP301L mice (Model 2508, Taconic Biosciences, Rensselaer, NY, USA) with neuron-specific expression of the most common FTDP-17 (frontotemporal dementia and parkinsonism linked to chromosome 17) mutation (Lewis et al., Nature Genetics 25, 402-405 (2000)).
(c) To generate Tg-TOMM6 mice, the cDNA of TOMM6 was inserted into the *Sall* site of the CosSHa.Tet vector (InPro Biotechnology Inc. San Francisco, CA, USA), which directs neuron-specific expression under control of the Syrian hamster prion protein (HaPrP) promoter (Hsiao et al., Science 274, 99-103 (1996), Scott et al., Protein Sci. 1, 986-997 (1992)). Plasmid sequences were removed by *NotI* digestion. The purified linear DNA (2ng/microL) was injected into fertilized oocytes dissected out from super-ovulated mice (FVB or B6 (C57BL/6J) mice). For DNA injection, oocytes were kept in M2 medium. After DNA injection, injected embryos were transferred into M16 medium and incubated overnight in M16 medium in the presence of 10 % CO₂ at 37°C in a humidified incubator. Thereafter, double-cell stage embryos were selected and transferred into the oviduct of pseudo-pregnant CD-1 foster mice. Pseudopregnancy was induced by overnight breeding with an infertile (vasectomized) male and detected by the presence of a vaginal plug. After birth, genomic DNA from F0 mice was isolated from ear punch biopsies taken at 3-4 weeks of age. Mice with stable genomic integration of the *TOMM6* transgene were identified by genotyping PCR and used for further breeding. Tg-CMV-TOMM6 mice with transgenic TOMM6 expression under control of the ubiquitous CMV immediate-early promoter/enhancer were also generated, which directs ubiquitous expression of a transgene (Fu et al., J. Biol. Chem. 288, 7738-7766 (2013)). For ubiquitous expression, the cDNA coding for TOMM6 was inserted into *EcoRI* and *XhoI* sites of pcDNA3.1 plasmid; Thermo Fisher Scientific, Waltham, MA, USA). Transgenic mice were generated as detailed above.
(d) As a model, which reproduces major features of sporadic AD and depression, 15 months-old male rats (Sprague Dawley) were aged according to the CUMS protocol for 4 weeks. The following stimuli were administered each week in a random order: two periods (7h and 17 h) of 45 ° cage tilt; soaked cage for 17 h; food deprivation (24h) and water deprivation (12h), twice a week; paired housing (17 h); overnight illumination during the dark phase, twice a week; noise (85 dB) in the room for 5h, twice a week; flashing light (60 flashes/min) for 6h, three times a week (AbdAlla et al., Biomed. Res. Int. 2015:917156 (2015); El-faramawy et al., Pharmacol. Biochem. Behav. 91, 339-344 (2009)). The sucrose preference test (2 % sucrose in water) was done immediately after a period of food and water deprivation. After four weeks of stress, more than 90 % of untreated stressed rats showed signs of anhedonia, which was documented by a decreased sucrose consumption in the sucrose preference test (≤ 50 % compared to non-stressed, age-matched control group and/or the stressed group treated with Compound-10F). As indicated, representative compounds (e.g. Compound-10F; 10 mg/kg body weight per day) were added to drinking water. The CUMS protocol was similarly performed with 12 months-old male Tg2576 mice for 3 months.
(e) Extracts and compounds: As indicated, extract P1 from *Galium aparine,* extract P3 from *Rubia cordifolia L.,* and small-molecule compounds (Compound-1, Compound-2, Compound-4, and Compound-7, Compound-10F: 10 mg/kg body weight/d) were added to drinking water. Compound-1 (alizarin; 1,2-dihydroxyanthraquinone) was purchased from Sigma (Sigma-Aldrich, St. Louis, MO, USA), and Compound-2 (pseudopurpurin, purpurin-3-carboxylic acid) was isolated from *Rubia tinctorum* according to established protocols (Richter D., Biochem. J. 31, 591-595 (1937); Derksen et al., Phytochem. Anal. 15, 397-406 (2004)). Synthesis of Compound-4, Compound-7 and Compound-10F is described below. Plant extracts were made by decoction of dried plant material for 1 h in boiling water (200 g pulverized dried plant material/1L water). For *in vivo* treatment, the plant extract was filtered, and the alizarin/anthraquinone content was adjusted to an amount of 0.05-0.1 mg/ml of alizarin by thin layer chromatography (TLC silica gel precoated plates 60F254 Merck, Darmstadt, Germany; mobile phase toluene/ethyl acetate/formic acid (70:25:5) or dichloromethane:methanol (9:1); visualization by visible light, UV 254 nm, 366 nm and/or by Bornträger reaction with KOH reagent (5 -10% KOH in methanol)). For incubation with HEK cells, the plant extract was further concentrated by rotary evaporation to a final volume of 10 ml, filtrated and added to the cell culture medium (20 micro-I/10 ml medium).
(f) Treatments: Treatment of the Tg2576 model was performed for six months without CUMS, or for three months (during the CUMS protocol), starting at an age of 12 months. Treatment of the Tg-TauP301L model was started at an age of 6 months and continued until 12 months. As a model, which reproduces major features of sporadic AD, aged 15 months-old male rats were subjected to the CUMS protocol for 4 weeks similarly as described (AbdAlla et al., Biomed. Res. Int. 2015:917156 (2015)). All mice/rats were kept on a light/dark cycle of 12 h light/12h dark, had free access to food and water (unless the CUMS protocol required a restriction) and were fed a standard rodent chow. At the end of the observation period, mice or rats were anesthetized (i.p.) with tribromoethanol (250 mg/kg) or urethane (1.2 g/kg), perfused intracardially with sterile PBS, and brains were isolated, and processed for histology or biochemical analyses. For protein extraction, hippocampi were dissected and immediately frozen in liquid nitrogen. All animal experiments were performed in accordance with NIH guidelines and approved by the local committees on animal experiments (University of Zurich Switzerland and Faculty of Veterinary Medicine, Cairo University).

### Animal model of atherosclerosis

ApoE-/- mice (Taconic Biosciences, Rensselaer, NY, USA) with deficiency of apolipoprotein E (ApoE-/-) due to disruption of the endogenous ApoE gene were used as a genetic model of atherosclerosis (Piedrahita et al., Proc. Natl. Acad. Sci. U.S.A., 89, 4471-4475, 1992). Atherosclerotic lesion area was determined by quantitative image analysis with oil red O-stained aortas. Total plasma cholesterol was determined by a commercial kit (Sigma). Mice were kept on a 12h dark and 12h light cycle, had free access to food and water, and were fed a standard rodent chow (AIN-95-based diet without vitamin E supplementation and with 7 % fat and 0.15 % cholesterol). For the study, we used ApoE-/- mice on a B6 (C57BL/6J) background and non-transgenic B6 control mice. Alizarin treatment (10 mg/kg/day in drinking water) was initiated at an age of 4 weeks and continued for eight months until the end of the observation period. At an age of 9 months, mice were anesthetized (ketamine/xylazine, 100/10 mg/kg) and perfused intracardially with sterile PBS. Aortas were isolated, rapidly dissected free of adipose tissue on ice and immediately frozen in liquid nitrogen or processed for further use.

### Clinical study in voluntary research participants

The study analyzed the treatment effect of topical administration of Compound-1 on warts. The study was performed according to the study protocol. Study design: The study was a phase-1, placebo-controlled study of Compound-1. The primary end-point was safety and tolerability. Secondary objectives were treatment effects on wart lesion size. The study included a cohort of 6 voluntary research participants (6 males; age 36 -72 years; all Caucasians) with diagnosed flat warts. A total number of 94 lesions were treated with Compound-1. Vehicle-treated warts (n=88) of each participant served as placebo group. Study participants received once daily topical applications of a Compound-1-containing hydrogel (0.2 %) or vehicle for 3 weeks. The study participants were under the medical supervision of Dr. Raafat Mahmood Fawzy (Clinical Director, Rabaa El Adaweya Medical Central Hospital, Cairo, Egypt). Clinical laboratory parameters of all research participants were within the normal range before, during the study and after completion of the study (observation period 4 weeks). There were no adverse effects in the study. Notably, there were no skin irritations, pain or other treatment-related adverse effects. The study protocol was conducted in accordance with the Declaration of Helsinki. All research participants provided written informed consent before participation. Research participants had the possibility to withdraw at any time from the study. But all participants wanted to complete the study.

### Compound synthesis

Compounds 3-10 and 10F were synthesized by EMC microcollections GmbH (Tübingen, Germany) and by ChiroBlock (Wolfen, Germany), according to established published protocols. Synthesis was performed by chemical synthesis methods, which were adapted from established protocols (for compound-3, compound-4, compound-5, compound-6: Singh et al., Organic Letters 14, 1198-1201 (2012); for compound-7, compound-8, compound-9, compound-10: Kaczanowska et al., J. Heterocyclic Chem. 48, 792 (2011), Kaczanowska et al., ACS Med. Chem. Lett. 1, 530-535 (2010)) and as detailed in Figs. 15D-G.

### Antibodies

The following antibodies were used for immunoblotting and immunohistology: Abeta plaques were stained with monoclonal mouse antibody BAM-10 (crossreactive with residues 1-12 of the Abeta peptide, Sigma-Aldrich, St. Louis, MO, USA); antibodies against AT2R (AGTR2) were raised in rabbit against an antigen encompassing amino acids 320-349 of the human AT2R (AGTR2) sequence (AbdAlla et al., J. Biol. Chem. 276, 39721-39726 (2001); J. Biol. Chem. 284, 6566-6574 (2009). HBsAg was detected with polyclonal antibodies raised against an antigen encompassing amino acids 1-20 of surface antigen HBsAg, and by a monoclonal HBsAg antibody produced in mouse (SAB4700767, Sigma-Aldrich, St. Louis, MO, USA). Suitable antibodies for detection of HBsAg expression, aggregation and monomerization were also isolated from human blood from individuals undergoing vaccination against Hepatitis B. Antibodies against TOMM6 were raised in rabbit against a recombinant human TOMM6 protein (HPA004801, Sigma-Aldrich, St. Louis, MO, USA); polyclonal antibodies were also raised in rabbit against a synthetic peptide encompassing amino acids 24-74 of human TOMM6 (ab205396; Abcam Cambridge, MA, USA). PHF-Tau was detected with monoclonal AT8 antibody (MN1020; Thermo Fisher Scientific, Waltham, MA, USA). Mouse monoclonal anti-NeuN antibody was raised against the neuron-specific protein NeuN (MAB377, clone A60, EMD Millipore, Merck KGaA, Darmstadt, Germany). Antibody against ATP6V1A was raised in rabbit against a recombinant fragment corresponding to amino acids 60-344 of human ATP6V1A (ab137574; Abcam, Cambridge, MA, USA).

### Immunohistochemistry

For Abeta plaque detection by immunohistology, paraffin-embedded brain sections (8 microm, 10-15 sections/brain taken at 30-50 microm intervals) were prepared from sham-treated Tg2576 mice (controls), and Tg2576 mice with 6 months of treatment with P3 extract and Compound-1, respectively. After antigen retrieval, sections were stained with monoclonal BAM-10 antibody (Sigma Aldrich, St. Louis, MO, USA), which cross-reacts with residues 1-12 of the Abeta peptide. Plaque burden was analyzed by computerized quantitative image analysis (AbdAlla et al., Int. J. Mol. Sci. 14, 16917-16942 (2013)). Hyperphosphorylated Tau was detected with AT8 antibody on paraffin-embedded brain sections from 12 months-old Tg-TauP301L mice (Model 2508, Taconic Biosciences, Rensselaer, NY, USA) without and with treatment with P3 extract, and Compound-1 for 6 months.

### Expression of HBsAg in yeast

Recombinant HBsAg cDNA (synthesized by GeneScript, Piscataway, NJ, USA) was inserted into the expression vector p42K-TEF (D3011001-1 Dualsystems Biotech AG, Zurich). Yeast transformants were selected in the presence of G418 in the yeast growth medium (YPD; Bacto-yeast extract 10 g/L, Bacto-peptone 20 g/L and D-Glucose 20 %). Yeast (Saccharomyces cerevisae) strain INVSc1 was used (Invitrogen GmbH, Nr. C810-00). After 3-4 days of cultivation to allow for protein expression, yeast cells were harvested by centrifugation, followed by washing with ice-cold PBS. The yeast cell pellet was immediately frozen at -70°C. Proteins were extracted for 30 min. at 4°C with solubilization buffer (1 % sodium deoxycholate, 0.1 % SDS, 0.1% NP40, 5 mM EDTA, 50 mM Tris, pH 8.0) supplemented with protease/phosphatase inhibitors (Cat. No. P8349, and/or PPC1010, Sigma-Aldrich, St. Louis, MO, USA). Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % mercaptoethanol), and 6 M urea for 90 min at room temperature. Proteins were stored at a concentration of 0.5 mg - 1 mg/ml at -70 °C for further use. Protein were separated by urea-containing SDS-PAGE (7.5-10 %) under reducing conditions, transferred to PVDF membranes by electrophoretic protein transfer in a tank transfer cell (Mini Trans-Blot cell, Bio-Rad GmbH, München, Germany), and transferred HBsAg proteins were detected in immunoblot with HBsAg-specific antibodies raised in rabbit against an N-terminal epitope of HBsAg, or by a monoclonal HBsAg antibody produced in mouse (SAB4700767, Sigma-Aldrich, St. Louis, MO, USA). Suitable antibodies for detection of HBsAg expression and aggregation were also isolated from human blood from individuals undergoing vaccination against Hepatitis B. Bound antibodies were visualized with F(ab)₂ fragments of enzyme-coupled secondary antibodies (Dianova GmbH, Hamburg, Germany), or by enzyme-coupled protein A (EMD Millipore, Merck KGaA, Darmstadt, Germany), and followed by enhanced chemiluminescent detection (ECL Prime, Amersham, GE Healthcare Life Sciences, Glattbrugg, Switzerland).

### Nano-LC-ESI-MS/MS

To isolate Compound-1-interacting hippocampal proteins, hippocampi were isolated from 5-10 aged Tg2576 mice, pulverized under liquid nitrogen and solubilized for 30 min at 4°C in solubilization buffer (1 % sodium deoxycholate, 0.05 % SDS, 0.05 % Tween 20 in PBS, pH 7.4, supplemented with protease inhibitors). Insoluble material was removed by centrifugation. The supernatant was diluted 1:5 in PBS (supplemented with protease inhibitors). To this solubilisate, Compound-1 was added at a concentration of 10 pM. Buffer exchange into 20 mM Tris pH 7.4 was performed by centrifugation over Amicon Ultra Centrifugal Filter Units (MWCO 3kDa; EMD Millipore Merck KGaA, Darmstadt, Germany). The concentrated proteins were dissolved in Laemmli sample buffer (Bio-Rad GmbH, München, Germany) supplemented with mercaptoethanol (355 mM) and subjected to 8% SDS-PAGE under reducing conditions. Compound-1-interacting proteins were identified by co-migration, protein bands were cut and subjected to nano-LC-ES-MS/MS (performed by Proteome Factory AG, Berlin, Germany). Proteins were identified using MS/MS ion search of the Mascot search engine (Matrix Science, London, England) and nr protein database (National Center for Biotechnology Information, Bethesda, MD). Ion charge in search parameters for ions from ESI-MS/MS data acquisition were set to '1+, 2+, or 3+'according to the common charge state distribution for the instrument and the method.

### Immunoblot detection of proteins and biochemical analyses

Immunoblot detection of PHF-Tau in the hippocampus of stressed rats subjected to the CUMS protocol was performed with monoclonal PHF (AT8) antibody and the guanidine-hydrochloride-extracted hippocampal protein fraction (6.25 M guanidine hydrochloride in 50 mM Tris, pH 8.0). Proteins were separated by 8% urea-containing SDS-PAGE under reducing conditions. SDS-insoluble hippocampal contents of Abeta1-40 and Abeta1-42 were determined by ELISA after serial tissue extraction in the presence of protease inhibitors according to the protocol of the manufacturer (Invitrogen KHB3481 and KHB3441).

For immunoblot detection of (hippocampal or HEK cell) proteins, e.g. Tomm6/TOMM6 and Atp6vla/ATP6V1A, (hippocampal) tissue was pulverized under liquid nitrogen, and HEK cell pellets were directly subjected to protein extraction. Proteins were extracted for 30 min at 4°C with solubilization buffer (1 % sodium deoxycholate, 0.1 % SDS, 0.1% NP40, 5 mM EDTA, 50 mM Tris, pH 8.0) supplemented with protease/phosphatase inhibitors (Cat. No. P8349, and/or PPC1010, Sigma-Aldrich, St. Louis, MO, USA). Particulate material was removed by centrifugation at 50 000 x g for 20 min at 4 °C. Solubilized proteins were concentrated and delipidated by precipitation with ice-cold acetone/methanol (12:2, final concentration 83 %) for 90 min at 4 °C. The pellet was dissolved in SDS-sample buffer supplemented with 2 % SDS, 0.1 M DTT (or 5 % mercaptoethanol), and 6 M urea for 90 min at room temperature. Proteins were stored at a concentration of 0.5 mg - 1 mg/ml at -70 °C for further use. Immunoblot detection of proteins was performed with affinity-purified antibodies or F(ab)₂ fragments of the respective antibodies after separation of proteins by urea-containing SDS-PAGE (7.5 % -10 %) under reducing conditions and electrophoretic protein transfer to PVDF membranes in a tank transfer cell (Mini Trans-Blot cell, Bio-Rad GmbH, München, Germany). Bound antibodies were visualized with F(ab)₂ fragments of enzyme-coupled secondary antibodies (Dianova GmbH, Hamburg, Germany) pre-absorbed to mouse serum proteins, or by enzyme-coupled protein A (EMD Millipore, Merck KGaA, Darmstadt, Germany) as applicable, and followed by enhanced chemiluminescent detection (ECL Plus, and/or ECL Prime, Amersham, GE Healthcare Life Sciences, Glattbrugg, Switzerland).

Proteins were also extracted from yeast cells and HEK cells expressing the indicated protein (i.e. HBsAg or AGTR2) as described above. Neuronal cell loss and hyperphosphorylated Tau in hippocampi of 15 months-old Tg2576 mice subjected to the CUMS protocol for 3 months was determined with crude homogenates of dissected hippocampi by direct binding assay with neuron-specific [¹²⁵I]-labeled anti-NeuN antibody (MAB377, clone A60, EMD Millipore, Merck KGaA, Darmstadt, Germany) and [¹²⁵I]-labeled AT8 antibody similarly as described (AbdAlla et al., J. Biol. Chem. 284, 6554-6565 (2009)).

### Pharmacokinetic and toxicology studies in rats and dogs

Sub-chronic toxicity studies in dogs (German Shepherd dogs) and rats (Sprague Dawley rats) were performed by the Center of Applied Analytical and Veterinary Studies, Cairo, Egypt.

### Measurement of serum level of alizarin (Compound-1), Compound-7 and Compound-10F in dogs and rats

Serum concentrations of alizarin were determined with serum isolated from the blood of male and female German shepherd dogs (age: 8-9 months) taken at different time points (0.5h, 1h, 2h, 4h, 6h, 8h, 24h) after oral intake of the indicated doses for 4 weeks. In addition, serum concentrations of alizarin (Compound-1) were determined in male and female Sprague Dawley rats (body weight: 200-250 g) treated for 1 month and/or three months by oral gavage of alizarin at a once daily dose of 100 mg/kg, 50 mg/kg, 25 mg/kg and 12.5 mg/kg. Serum proteins were removed by acetonitrile precipitation, and alizarin was extracted by chloroform before separation on an HPLC-C18 column (Poroshell 120 EC-C18, 3.0x50 mm, Agilent) with an HPLC system (Agilent 1100 Series) and detection at OD245 nm. For quantitative determination of Compound-7 and Compound-10F in rat and dog serum, serum proteins were also removed by acetonitrile precipitation before HPLC analysis as detailed above and detection at OD254 nm.

### Fluorescence spectrophotometry of AT2R-Cer and AT1R-Cer expressed in HEK cells

AT2R-Cer (AGTR2-Cerulean) is a fusion protein, in which full-length AGTR2 is fused C-terminally with a 5-Gly linker to the Cerulean variant (Cer) of the enhanced green fluorescent protein. The analogous construct of AGTR1-Cer, in which full-length AGTR1 is fused C-terminally with a 5-Gly linker to the Cerulean variant (Cer) of the enhanced green fluorescent protein, was generated in frame of a previous study (Quitterer et al., Biochem. Biophys. Res. Commun. 409, 544-549 (2011)). AGTR2-Cer and AGTR1-Cer were expressed in HEK cells with and without *TOMM6* (cDNA coding for TOMM6 was inserted into *EcoRI* and *XhoI* sites of pcDNA3.1 plasmid; Figure 1). Thirty-six to forty-eight hours after transfection, cells were detached by 0.05 % Trypsin-EDTA solution, trypsin-EDTA was rapidly removed by washing with PBS, cells were collected by centrifugation and suspended in incubation buffer (335 mg KCI, 294 mg CaCl2, 407 mg MgCl2, 8.2 g NaCl, 2.4 g HEPES-Na+ ad 1000 ml H2O, pH 7.4). Cells were suspended at a concentration of 0.5 - 1 x 10 6 cells /ml. The AT2R-Cer and AT1R-Cer fluorescence were recorded in a fluorescence spectrometer (Perkin Elmer LS50B: Perkin Elmer, Waltham, MA, USA) at an excitation wavelength of 420 nm and emission between 440-600 nm. Peak fluorescence intensity at 495 nm was determined in the absence and presence of *TOMM6* co-expression or TOMM6 stabilizer/inducer/activator.

### Whole genome microarray gene expression analysis of aortic genes

Whole genome microarray gene expression profiling was performed with aortic specimens isolated from 9-month-old ApoE-/- mice without treatment and with oral treatment for 8 months with alizarin (10 mg/kg/day), and untreated age-matched B6 control mice. Total RNA was isolated by the RNeasy Mini kit according to the protocol of the manufacturer (Qiagen). RNA purity was between 1.8 and 2 as determined by the absorbance ratio of 260/280. RNA quality and absence of signs of degradation were also controlled by RNA electrophoresis on a denaturing agarose gel by the presence of bright bands of 28S and 18S ribosomal RNA. Total RNA was processed for whole genome microarray gene expression profiling with the GeneChip One-Cycle Target Labeling System (Affymetrix) according to the protocol of the manufacturer (Affymetrix GeneChip Expression Analysis Technical Manual Rev. 5). Hybridization with the GeneChip (Mouse genome MG430 2.0 Array; Affymetrix) was done with 15 microg of fragmented cRNA in 200 microl of hybridization solution in a Hybridization Oven 640 (Affymetrix) at 45 °C for 16h. Washing and staining of gene chips was done with the Affymetrix Fluidics Station 450 followed by scanning (Affymetrix GeneChip Scanner 7G). Signal processing was performed with GCOS (v. 1.4. Affymetrix). Data were scaled to a target value of 300. Probe sets (with call present and/or signal intensity ≥100) with significantly different signal intensity (p<0.05) indicative of different gene expression between different groups were identified by TIGR MultiExperiment Viewer (MeV v4.9).

### Example 2: Identification of Tomm6 as an interacting/stabilizing protein of Compound-1, Compound-2, and extract P1 and P3 from Galium aparine and Rubia cordifolia

The major target of the prototypical anthraquinone, Compound-1 (alizarin) was searched for. To this end, a solubilisate of hippocampal proteins from aged Tg2576 mice supplemented with 10 microM Compound-1 was prepared, and the mixture was separated by SDS-PAGE under reducing conditions. The Compound-1-interacting proteins were identified by co-migration, cut from the gel, and subjected to protein identification. Nano-LC-ESI-MS/MS analysis identified Tomm6 (Mitochondrial import receptor subunit TOMM6 homolog) as a major Compound-1-interacting protein (Figure 1A).

TOMM6 (Tom6) is an essential component of the TOM machinery, i.e. the multisubunit translocases in the outer mitochondrial membrane, which are required for the import of nucleus-encoded precursor proteins. It was investigated whether treatment with Compound-1, Compound-2, extract P1 and P3 from *Galium aparine* and *Rubia cordifolia* mediated protein stabilization of Tomm6 in the hippocampus of Tg2576 mice. Aged Tg2576 mice were treated for 6 months with Compound-1, Compound-2, P1 and P3 extract. Hippocampal protein contents of Tomm6 were determined in immunoblot. Immunoblot detection showed that the hippocampal protein content of Tomm6 in Tg2576 mice was substantially increased by treatment with Compound-1 (alizarin), Compound-2 (pseudopurpurin), P1 extract from *Galium aparine,* and P3 extract from *Rubia cordifolia* (Figure 1B,C). Thus, two prototypical anthraquinones from the *Galium* and *Rubia* genera, alizarin and pseudopurpurin, and extracts from two different anthraquinone-containing genera, *Galium* and *Rubia,* mediated stabilization/induction of the Tomm6 protein *in vivo,* in the hippocampus of aged Tg2576 AD mice.

### Example 3: TOMM6 enhances AT2R protein folding in human cells

It was found that Compound-1, Compound-2, extract P1 and extract P3 from the *Galium* and *Rubia* genera of the *rubiaceae* family interacted with and stabilized TOMM6. Next, it was analyzed whether stabilization/induction of TOMM6 was sufficient to exert cell/neuro-protection and prevented protein aggregation/misfolding of the AT2R (AGTR2) in HEK cells. Misfolded and aggregated AT2R promotes neurodegeneration whereas the intact AT2R could exert neuroprotection in AD models and patients (AbdAlla et al., J. Biol. Chem. 284, 6554-6565 (2009); AbdAlla et al., J. Biol. Chem. 284, 6566-6574 (2009); Quitterer and AbdAlla, Pharmacol. Res. pii: S1043-6618(18)31950-9 (2019)).

To analyze the effect of an increased cellular TOMM6 protein, the cDNA of *TOMM6* was inserted into the EcoRI and Xhol sites of the expression plasmid pcDNA3.1, which enables transient and stable expression of a cDNA under control of the ubiquitous CMV immediate-early promoter/enhancer in cells and in vivo (Figure 2A,B). The AT2R-Cerulean (AT2R-Cer) was generated by fusion of the AT2 receptor at the carboxyl-terminus with Cerulean similarly as described for the AT1R-Cer construct (Quitterer et al., Biochem. Biophys. Res. Commun. 409, 544-549 (2011)). The Cerulean variant of the ECFP was used because Cerulean is 2.5-fold brighter than the cognate ECFP (Rizzo et al., Nat. Biotechnol. 22, 445-449 (2004)).

Upon expression in HEK cells, the total amount of cell membrane-localized AT2R-Cer was quantified in a fluorescence spectrophotometer. The co-expression of TOMM6 led to a signifycantly increased AT2R-Cer-specific fluorescence intensity, which is indicative of improved AT2R-Cer protein folding (Figure 3A).

In contrast to AT2R-Cer, the related AT1R-Cer (AGTR1-Cerulean) was not increased by TOMM6 but AT1R-Cer was significantly decreased by TOMM6 (Figure 3B). This finding is significant because the AT1R (AGTR1) protein is enhanced by reactive oxygen species, ROS (Banday et al., Hypertension 57, 452-459 (2011)). Moreover, an increased expression/activation of AT1R contributes to neurodegeneration and increases Alzheimer pathology *in vivo* (AbdAlla et al., Int. J. Mol. Sci. 14, 16917-16942 (2013); AbdAlla et al., Biomed. Res. Int. 2015:917156 (2015); Quitterer and AbdAlla, Pharmacol. Res. pii: S1043-6618(18)31950-9 (2019)). Taken together, these cellular data are compatible with the notion that an increased TOMM6 level reduces the cellular ROS content by improving the function of mitochondria, which is the major inducer of cellular ROS. This process of "mitochondrial healing" could also be exerted by the stabilization of TOMM6 with the prototypical anthraquinones Compound-1 (alizarin), Compound-2 (pseudopurpurin), and the anthraquinone-containing plant extracts P1 and P3 from *Galium aparine* and *Rubia cordifolia.* As a consequence of "mitochondrial healing", redox-sensitive folding of AGTR2 is improved whereas folding of AGTR1 is inhibited.

### Example 4: Generation of Tg-TOMM6 mice

The above data showed that increased protein levels of TOMM6 promoted the folding of the AT2R protein in human cells, which is misfolded and aggregated in brains of AD patients with sporadic AD (AbdAlla et al., J. Biol. Chem. 284, 6554-6565 (2009)). In addition, TOMM6 reduced the protein level of neurotoxic AT1R. To further validate the neuroprotective function of TOMM6 *in vivo,* transgenic mice with expression of *TOMM6* under control of the neuron-specific hamster prion protein (HaPrP) promoter were generated. To generate Tg-TOMM6 mice, the cDNA of TOMM6 was inserted into the *Sall* site of the CosSHa.Tet vector (Figure 4A), which directs neuron-specific expression under control of the Syrian hamster prion protein (HaPrP) promoter (Hsiao et al., Science 274, 99-103 (1996); Scott et al., Protein Sci. 1, 986-997 (1992); InPro Biotechnology Inc. San Francisco, CA, USA). Plasmid sequences were removed by *NotI* digestion. The purified linear DNA (2ng/microL) was injected into fertilized oocytes dissected out from super-ovulated FVB or B6 (C57BL/6J) mice. For DNA injection, oocytes were kept in M2 medium. After DNA injection, injected embryos were transferred into M16 medium and incubated overnight in M16 medium in the presence of 10 % CO₂ at 37°C in a humidified incubator. Thereafter, double-cell stage embryos were selected and transferred into the oviduct of pseudo-pregnant CD-1 foster mice. Pseudopregnancy was induced by overnight breeding with an infertile (vasectomized) male and detected by the presence of a vaginal plug. After birth, genomic DNA from F0 mice was isolated from ear punch biopsies taken at 3-4 weeks of age. Mice with stable genomic integration of the *TOMM6* transgene were identified by genotyping PCR and used for further breeding.

To generate double-transgenic Tg-2576-TOMM6 mice, homozygous Tg-TOMM6 mice were crossed with heterozygous Tg2576 AD mice and double-transgenic Tg2576-TOMM6 mice were identified by genotyping PCR (sequences of primers used for genotyping PCR are derived from TOMM6-flanking CosSHa.Tet vector sequences and from the TOMM6 cDNA sequence) (Figure 4B).

### Example 5: Transgenic TOMM6 expression retards major neuropathological features in the hippocampus of Tg2576 AD mice

The goal was to investigate whether an increased TOMM6 level was sufficient to retard neurodegeneration in the Tg-2576 AD model. Aged 18 months-old double-transgenic Tg2576-TOMM6 mice had an increased hippocampal protein level of TOMM6 as determined in immunoblot compared to single-transgenic Tg2576 AD mice (Figure 5A).

Double-transgenic Tg-2576-TOMM6 mice were used and it was analyzed whether an increased hippocampal TOMM6 protein level affected the neuropathological features in double-transgenic Tg2576-TOMM6 mice compared to single-transgenic Tg2576 controls. Hippocampal contents of SDS-insoluble Abeta1-40 and Abeta 1-42 were significantly decreased in double-transgenic Tg2576-TOMM6 mice compared to single-transgenic Tg2576 controls (Figure 5B,C). Thus, an increased hippocampal TOMM6 protein level is sufficient to retard the development of a major neuropathological feature of AD, i.e. the accumulation of SDS-insoluble Abeta peptides in the hippocampus of Tg2576-TOMM6 mice.

As detailed before, the Tg2586 AD model is largely devoid of major symptoms of clinical AD, i.e. neuronal loss and gross memory impairment (AbdAlla et al., J. Biol. Chem. 284, 6566-6574 (2009); Ashe and Zahs, Neuron 66, 631-645 (2010)). To enhance the process of neurodegeneration in Tg2576 AD mice, we subjected Tg2576 mice to chronic mild environmental stress (AbdAlla et al., J. Biol. Chem. 284, 6554-6565, (2009); Briones et al., Br. J. Pharmacol. 165, 897-907 (2012)), which is known to aggravate symptoms of dementia and neurodegeneration in patients (Peavy et al., Biol. Psychiatry 62, 472-478 (2007); Wilson et al., Neuroepidemiology 27, 143-163 (2006)). Quantitative analysis detected hyperphosphorylated PHF-Tau in the hippocampus of 15 months-old Tg-2576 mice after 3 months of CUMS (Figure 5D). The presence of increased neuronal TOMM6 in double-transgenic Tg2576-TOMM6 mice led to a significantly decreased hippocampal content of hyperphosphorylated PHF-Tau in 15 months-old Tg2576-TOMM6 mice as determined by direct binding assay with [¹²⁵I]-labeled AT8 antibody, which is specific for PHF (paired helical filament) form of hyperphosphorylated Tau (Figure 5D).

Concomitantly, hippocampal neuronal loss was also prevented by transgenic TOMM6 expression, as determined by direct binding assay with [¹²⁵I]-labeled anti-NeuN antibody (Figure 5E). These findings show that an increased hippocampal protein level of TOMM6 is sufficient to promote neuroprotection against major neuropathological features in the hippocampus of Tg2576 AD mice, i.e. accumulation of insoluble Abeta peptides, formation of hyperphosphorylated PHF-Tau and overt neuronal loss.

### Example 6: Identification of TOMM6-interacting (inducing) compounds without antioxidant activity (Figure 6 and Figure 7)

TOMM6-inducing compounds were searched for and a screening system was established in HEK cells. HEK cells were cultured in the absence and presence of a test compound at a final concentration of 20 microM. Cells were harvested, proteins were extracted and TOMM6 protein levels were determined in immunoblot with TOMM6-specific antibodies. This assay identified compound 3 - 10 as TOMM6-interacting compounds and inducers of TOMM6 (Figure 6 and Figure 7). The following TOMM6-interacting (inducing) compounds were identified: Compound-3: N-[(3-chlorophenyl)methyl]-2-(3-piperidyl)oxazole-4-carboxamide; Compound-4: N-[(3,4-dichlorophenyl)methyl]-2-(3-piperidyl)oxazole-4-carboxamide; Compound-5: 2-[(1S)-1-amino-2-methyl-butyl]-N-[(3,4-dichlorophenyl)methyl]oxazole-4-carboxamide; Compound-6: 2-[(1S)-1-amino-2-methyl-propyl]-N-[(3,4-dichlorophenyl)methyl]oxazole-4-carboxamide; Compound-7: 6-(aminomethyl)-4-(4-chlorophenyl)-2-methyl-pyridine-3-carboxamide; Compound-8: 4-(2,4-dichlorophenyl)-6-[(1,3-dioxoisoindolin-2-yl)methyl]-2-methyl-pyridine-3-carboxamide; Compound-9: 4-(4-chlorophenyl)-6-[(1,3-dioxoisoindolin-2-yl)methyl]-2-methyl-pyridine-3-carboxamide; and Compound-10: 4-(4-chlorophenyl)-6-(hydroxymethyl)-2-methyl-pyridine-3-carboxamide).

### Example 7: TOMM6-interacting (inducing) Compound-4 and Compound-7 inhibit symptoms of AD and neurodegeneration in AD model mice

The TOMM6-interacting (inducing) Compound-4 and Compound-7 were selected for in vivo testing. Treatment with Compound-4 and Compound-7 led to a strong increase in hippocampal Tomm6 protein content of Tg2576 AD mice subjected to the CUMS protocol (Figure 8A). In addition, Compound-4 and Compound-7 inhibited hippocampal accumulation of aggregated Abeta1-40 and Abeta1-42 in aged 18-month-old Tg2576 AD mice (Figure 8B). Treatment with Compound-4 and Compound-7 also decreased hippocampal PHF tau hyperphosphorylation (Figure 8C) and retarded overt neuronal loss in Tg2576 mice subjected to chronic mild stress with the CUMS protocol (Figure 8D). Together these experiments show that treatment with TOMM6-inducing compounds retard major symptoms of AD and neurodegeneration in vivo.

### Example 8: Emodin in vivo tests.

Treatment with emodin (6-methyl-1,3,8 trihydroxyanthrachinone; formula is shown in Figure 9 C in comparison to Compound-1 and Compound-2 (A)) at a dose of 100 mg/kg/day, for 4 weeks showed side effects and led to a decrease in body weight of 12-week-old B6 mice compared to untreated controls (Fig. 10A). The decreased body weight could be due to the laxative effect of emodin and/or decreased food intake (Abu Eid et al., Eur. J. Pharmacol. 798, 77-84, 2017). In agreement with this statement, the daily food intake during emodin treatment was lower compared to untreated B6 control mice (Fig. 10B). In contrast to emodin, treatment with equivalent doses of alizarin (Compound-1) and pseudopurpurin (Compound-2) did not show side effects (Fig. 10A,B). In contrast to emodin, treatment of B6 mice with alizarin at an oral dose of 100 mg/kg/day for 1 month did not significantly alter body weight and food consumption (Fig. 10A,B). Similarly, treatment of mice for 1 month with pseudopurpurin at an oral dose of 100 mg/kg/day did not significantly alter body weight and food consumption (Fig. 10A,B). In agreement with these data, another study found that pseudopurpurin was not toxic when added as a food supplement, i.e. 0.5% pseudopurpurin was added to diet, which corresponds to a daily intake of pseudopurpurin of ~ 250 mg/kg/day (Wu et al., Int. J. Mol. Sci. 13, 3431-3443, 2012). Taken together, disclosed Compound-1 (alizarin) and Compound-2 (pseudopurpurin) are Tomm6/TOMM6 inducers in cells and in vivo and exert neuroprotection in different models of neurodegenerative diseases, e.g. familial AD, sporadic AD and tauopathy. In contrast to alizarin and pseudopurpurin, the laxative anthrachinone, emodin, has some adverse effects.

### Example 9: Structural modifications of alizarin.

Glycosylated derivatives of alizarin and pseudopurpurin can be isolated from alizarin- and pseudopurpurin-containing plants, and also can be synthesized, e.g. with bio-catalysed reactions applying glycosyltransferases (Ati et al., Beilstein J. Org. Chem. 13, 1857-1865, 2017). In addition, alizarin and other anthrachinone derivatives can be fed to recombinant E. coli as a substrate for biotransformation to generate alizarin-glucosides (Nguyen et al., Molecules 23, 2171, 2018). Alizarin is not a tumor promotor in mammalian cells and in vivo (Westendorf et al., Mutat. Res. 240, 1-12, 1990; Marec et al., Planta Med. 67, 127-131, 2001; Wölfle et al., Cancer Res. 50, 6540-6544, 1990; Poginsky et al., Carcinogenesis 12, 1265-1271, 1991). However, when tested in the Ames test with different Salmonella strains, alizarin could be mutagenic in the Salmonella strain TA1537 (Westendorf et al., Mutat. Res. 240, 1-12, 1990; Liberman DF et al., Applied and Environmental Microbiology 43, 1354-1359, 1982) and strain TA2637 (Tikkanen et al., Mutat. Res. 116, 297-304, 1983). The mutagenicity in bacteria is related to the hydroxyl groups of alizarin (Tikkanen et al., Mutat. Res. 116, 297-304, 1983). Anthrachinones with halogen substituents are not mutagenic (Brown JP and Brown RJ, Mutat. Res. 40, 203-224, 1976). Therefore, to minimize the potential risk of mutagenicity, the present invention in general and for all embodiments encompasses the use of all halogen- (e.g., F, Cl, Br) -substituted compounds described herein, preferably of alizarin and pseudopurpurin, more preferably fluorine-substituted alizarin and pseudopurpurin and/or mimetics of alizarin and pseudopurpurin (see Fig. 10C).

### Example 10: Medical uses of alizarin for supplementary treatment and prevention of Hepatitis B virus infection, and treatment and prevention of human papilloma virus (HPV) infection

The present invention encompasses the medical use of the compounds claimed herein for preventive and supportive treatment of Hepatitis B infection because it was surprisingly found that not only plant extracts P1 and P2 but also their active isolated ingredient alizarin (Compound-1) inhibited the aggregation and assembly of HBsAg (Fig. 10D). HBsAg aggregation and assembly is required and mandatory for HBV virion formation and HBV infection (Venkatakrishnan and Zlotnick, Annu. Rev Virol. 3, 429-451, 2016). The treatment modality of the present invention in general and for all embodiments could supplement current antiviral treatment regimens of Hepatitis B infection, which include according to WHO guidelines potent nucleos(t)ide analogues with high barrier to resistance, i.e. entecavir, tenofovir disoproxil, and tenofovir alafenamide. Nevertheless, chronic persistent Hepatitis B infection, which affects ~240 million patients worldwide cannot be cured and accounts for ~650 000 deaths/year worldwide (WHO publication, Guidelines for the prevention, care and treatment of persons with chronic hepatitis B infection, March 2015).

The present invention in general and for all embodiments also encompasses the medical use of the compounds described herein, preferably alizarin (Compound-1), for prevention and treatment of human papilloma virus (HPV) infections, in particular, because it was found that 3 weeks of once daily topical application of compounds according to the present invention, e.g. a Compound-1-containing hydrogel (0.2 %), are an effective treatment for HPV-induced warts (see Fig. 10E showing the results obtained from 6 different voluntary research participants diagnosed with flat warts compared to the placebo (Fig. 10E)). A total number of 94 lesions were treated with Compound-1. Vehicle-treated warts (n=88) served as placebo. All treated warts showed a response. After treatment with Compound-1, more than 86 % of all lesions showed a complete response (100 % decrease of the lesion area) and 13.8 % showed a partial response (> 50 % - < 100 % decrease of lesion area) whereas only 2.2 % and 7.9 % of the placebo-treated lesions (treated with vehicle) showed a complete or partial response, respectively (Fig. 10E). The treatment effect of Compound-1 against HPV infection is directly related to its activity as TOMM6 inducer/activator, which is a sufficient cause to inhibit (amyloidogenic) protein aggregation in cells and in vivo. In this context, HPV infection is another disease with causal involvement of an aggregation-prone amyloidogenic protein. Without wishing to be bound by theory, it is believed that Infections with human papillomaviruses (HPV) rely on the highly amyloidogenic protein E1^E4, also known as the HPV E4 protein (McIntosh et al., 82, 8196-8203 (2008)). The HPV E4 protein, which uses the amyloid-fold as does amyloid-beta, is the most abundantly expressed viral transcript/protein in HPV-infected epithelia, and mediates HPV-mediated disruption of cellular integrity, HPV release from infected epithelial cells and virus transmission (McIntosh et al., 82, 8196-8203 (2008); Doorbar, Virology 445, 80-98 (2013)). In HPV-induced warts, the aggregation-prone HPV E4 protein can constitute between 20 % and 30 % of the total wart protein (Doorbar et al., EMBO J. 5, 355-362 (1986)). The expression of E1^E4 relies on self-aggregation of the HPV E4 protein (Bryan et al., Virology 241, 49-60 (1998)).

Common warts greatly affect the quality of life of the patients, which can be attributed to the persistence and recurrence of warts. Malignant transformation usually develops in patients with genital warts and immunocompromised patients. Several high-risk HPV subtypes are associated with malignancies (e.g. HPV type 6, 11, 16, 18, 31, 35) and HPV type 5, 8, 20 and 47. This causal relationship between HPV infection and cancer raises concerns about the association between persistent HPV infection and the neoplastic progression of common warts to non-genital cancers, e.g. skin cancer (Lipke, 4, Clinical Medicine and Research, 273-293 (2006)). Warts are common and affect about 10 % of the population worldwide. Currently, there is no cure for HPV infection and available treatments aim to eliminate symptoms. Treatment approaches include minor surgery, laser surgery, cryotherapy, peeling agents such as salicylic acid, medications (e.g. bleomycin, and imiquimod, which is an immunotherapy). Most treatment approaches are painful such as surgery, cryotherapy, laser therapy, and/or cause side effects such as burning and stinging and even may leave a scar. Even with treatment, warts tend to recur or spread. Therefore, there is an urgent need to improve the therapy and/or develop a new treatment approach for HPV.

### Example 11: Alizarin (Compound-1) retards the development of atherosclerosis in ApoE-deficient mice as a model of atherosclerosis.

Alzheimer's Disease (AD) is the most frequent form of dementia in the elderly. The second commonest form of dementia is vascular dementia (Appleton et al., Clin. Sci 131, 1561-1578, 2017). Often vascular dementia and AD coexist (ladecola, Neuron 80, 844-866, 2013). Atherosclerosis and ensuing cardiovascular disease are major risk factors for vascular dementia (Javanshiri et al., J. Alzheimers Dis. 65, 1247-1258, 2018). Cardiovascular risk factors are also considered to enhance AD pathology (Tublin et al., Circ. Res. 124, 142-149, 2019). Notably, atherosclerotic vascular lesions are causally related to the pathogenesis of vascular dementia, mixed forms of vascular dementia and even AD (Li et a., Chin. Med. J. (Engl) 131, 471-476, 2018). Cholesterol-lowering statin use reduces the risk of dementia in patients with stroke (Pan et al., J. Stroke Cerebrovasc. Dis. 27, 3001-3007, 2018). These data clearly indicate that atherosclerotic vascular disease is a contributor to dementia. However, there is an increasing concern that cholesterol-lowering treatment could enhance or even cause cognitive problems (Li et a., Chin. Med. J. (Engl) 131, 471-476, 2018). In view of this concern, it was investigated whether treatment with alizarin, which counteracts AD pathology, could interfere with atherosclerotic lesion development without altering plasma cholesterol levels. Alizarin (Compound-1) is a TOMM6/Tomm6 inducer, and TOMM6 induction restores the function of the angiotensin II type-2 receptor, AGTR2. Restoration of AGTR2 could counteract atherosclerosis because a previous study showed that inhibition/deficiency of AGTR2 in ApoE-deficient (ApoE-/-) mice as a model of atherosclerosis enhances atherosclerosis progression whereas AGTR2 stimulation counteracts atherosclerosis, in part by inhibition of the pro-atherogenic AGTR1 (Iwai et al., Circulation 112, 1636-1643, 2005). To investigate the impact of alizarin treatment on atherosclerosis, ApoE-/- mice were used, which accumulate substantial atherosclerotic lesions in the aorta at an age of 8-10 months. ApoE-/- mice were treated for 8 months with alizarin (10 mg/kg/d in drinking water) starting at an age of 4 weeks. Atherosclerotic lesion area was assessed with oil red O-stained aortas isolated from 9-month-old ApoE-/- mice (Fig. 11A). Alizarin treatment led to a significantly decreased atherosclerotic lesion area, which was 3.6-fold lower compared to that of untreated ApoE-/- mice (Fig. 11A). As a control, alizarin treatment did not alter the increased plasma cholesterol level of ApoE-/- mice (Fig. 11B). Whole genome gene expression analysis was performed to identify atherosclerosis-related genes altered by alizarin treatment. It was found that alizarin treatment led to a significantly decreased expression of the aortic chemokine receptor 9, *Ccr9* (Fig. 11C), which is a pro-atherogenic cytokine receptor and enhances atherosclerotic lesion development (Abd Alla et al., J. Biol. Chem. 285, 23496-23505, 2010). It was further investigated whether alizarin treatment was neuroprotective and retarded the atherosclerosis-induced degeneration of vascular nerves. Vascular neurodegeneration and autonomic neuropathy are major pathologic factors of atherosclerosis (Meyer et al., Diabet. Med. 21, 746-751, 2004; Abd Alla et al., Front. Physiol. 4, 148, 2013). Atherosclerosis-induced neurodegeneration of vascular neurons in ApoE-/- mice is documented by decreased aortic expression levels of neuronal marker genes (Abd Alla et al., Front. Physiol. 4, 148, 2013). The aortic expression levels of neuronal marker genes of alizarin-treated ApoE-/- mice were determined compared to untreated ApoE-/- mice and untreated B6 mice without atherosclerosis (Fig. 11D,E). It was found that the aortic expression levels of typical neuronal marker genes, neuropeptide Y (*Npy*) and synaptosomal nerve-associated protein 25 (*Snap25*), were down-regulated by atherosclerosis in ApoE-/- mice (Fig. 11D,E). In contrast, in alizarin-treated ApoE-/- mice the aortic expression levels of neuropeptide Y (Npy) and synaptosomal nerve-associated protein 25 (Snap25) were not down-regulated and maintained at the levels of the non-transgenic B6 controls (Fig. 11D,E). These data show that alizarin treatment not only retards the development of atherosclerotic lesions but also prevents the atherosclerosis-induced degeneration of vascular neurons. Hence, the present invention in general and for all embodiments also encompasses the medical use of the compounds described herein, preferably alizarin (Compound-1) and the TOMM6/Tomm6-inducing compounds, for the prevention and treatment of atherosclerosis, atherosclerosis-induced neurodegeneration, diabetic neuropathy and autonomic neuropathy (for which atherosclerosis are major risk factors; Meyer et al., Diabet. Med. 21, 746-751, 2004) and other atherosclerosis-induced diseases, e.g. stroke, vascular dementia, coronary artery disease, myocardial infarction.

### Example 12: Pharmacokinetic characterization of alizarin (Compound-1) in dogs and rats.

In view of the beneficial effects of alizarin (Compound-1) in murine AD and atherosclerosis animal models and the absent toxicity in the therapeutic dose range, further in vivo characterization of alizarin (Compound-1) was performed in dogs as a non-rodent animal model (Fig. 12). Measurement of alizarin serum level in dogs showed a peak serum concentration at t=1 h after repeated once daily oral intake of alizarin at a dose of 2 mg/kg/day and 6 mg/kg/day (Fig. 12A-C). A second alizarin serum peak was observed at t=4h after oral intake (Fig. 12B). The second peak could reflect enterohepatic recirculation of alizarin. A previous study, which determined alizarin plasma level in rats, also found two alizarin peaks (Gao et al., J. Sep. Sci. 41, 2161-2168, 2018). The peak serum concentrations in dogs after repeated oral intake of a once daily alizarin dose of 2 mg/kg and 6 mg/kg were 0.146±0.017 microg/ml and 0.467±0.022 microg/ml (Fig. 12D). Treatment of dogs for four weeks with alizarin (Compound-1) at a once daily oral dose up to 6 mg/kg/d did not induce any detectable adverse effects regarding behaviour, food intake, weight gain, cardiovascular function parameters (i.e. blood pressure, heart rate, ECG), number of circulating red and white blood cells, and clinical chemistry parameters for liver and kidney function. Pharmacokinetic data of alizarin were also determined in rats after repeated oral gavage. It was found that the oral bioavailability of alizarin in dogs was much higher than in rats because the oral alizarin doses required to achieve equivalent peak serum concentrations were approximately 10-fold higher in rats compared to dogs (Fig. 12D). This observation is in agreement with the equivalent surface area dosage rule, according to which rats and mice usually require a 10-12-fold higher dose than dogs and humans. Taken together, oral intake of alizarin achieves therapeutic serum concentrations in rats and dogs.

### Example 13: Sub-chronic toxicity study of alizarin in rats documents a wide therapeutic index.

The oral toxicity of alizarin (Compound-1) in rats was determined in a sub-chronic toxicity study. Rats were treated for 3 months with a once daily oral dose of alizarin of 25 mg/kg/day, 50 mg/kg/day and 100 mg/kg/day. The study did not find any significant adverse effects of alizarin up to a daily dose of 100 mg/kg/day regarding body weight, liver, heart and kidney weight, serum levels of calcium, creatinine, blood urea nitrogen (BUN), alanine transaminase (ALT) and aspartate aminotransferase (AST) (Fig. 13A-I). Histopathology analysis of major organs (heart, liver, kidney) did not show any detectable alterations (Fig. 13J). These findings indicate a wide therapeutic index of alizarin in vivo with a more than 10-fold difference between therapeutic and toxic doses because neuroprotective effects of alizarin in rodent animal models of AD and atherosclerosis were seen at a once daily dose of 10 mg/kg. The good tolerability of alizarin in rats and dogs is supported by previous observations in humans, which document that long-term treatment for several years with alizarin and alizarin-containing plant extracts is not toxic and was previously used to prevent recurrence of calcium-containing urinary stones (Lorenz et al., Methods Find. Exp. Clin. Pharmacol. 7, 637-643, 1985). Taken together, alizarin has good oral bioavailability and a wide therapeutic window.

### Example 14: Pharmacokinetic data of Compound-7 in dogs and rats.

Pharmacokinetic data of Compound-7 were determined as another representative TOMM6/Tomm6 inducer with a different chemical structure than alizarin (Compound-1). In dogs, Compound-7 showed peak serum concentrations of 3.96 microg/ml, 1.56 microg/ml and 0.59 microg/ml measured at t=1h after drug intake after repeated once daily oral dosing of 12.5 mg/kg, 5 mg/kg and 2 mg/kg (Fig. 14A,B). The oral bioavailability of Compound-7 in dogs was much higher than in rats because oral doses of Compound-7 required to achieve equivalent peak serum concentrations were approximately 10-fold higher in rats compared to dogs (Fig. 14B). Again, this observation is in agreement with the equivalent surface area dosage rule, according to which rats and mice usually require a 10-12-fold higher dose than dogs and humans. In rats, the peak serum concentration after repeated once daily oral dosing was achieved at t=1h (Fig. 14C). Analogously to alizarin (Compound-1), a second serum peak of Compound-7 was observed in rats at t=3h after oral intake (Fig. 14C). This second peak could reflect enterohepatic recirculation of Compound-7. Together these data show that Compound-7 has oral bioavailability in rats and dogs. Compound-7 did not show any detectable toxic side effects up to an oral dose of 12.5 mg/kg/day in dogs and up to an oral dose of 50 mg/kg/day in rats during four weeks of treatment. At a daily oral dose of 100 mg/kg/d in rats, a slightly decreased daily food intake was observed. These findings indicate a wide therapeutic index of Compound-7 in vivo with an almost 10-fold difference between therapeutic and toxic doses because neuroprotective effects of Compound-7 in rodent animal models of AD and neurodegeneration were seen at a once daily oral dose of 10 mg/kg/d (cf. Fig. 8).

### Example 15: Development of Compound-10F.

A series of compounds (i.e. Compounds 7-10) with a common 4-phenyl-2-methyl-pyridine-3-carboxamide backbone was identified as TOMM6/Tomm6 inducers (cf. Fig. 7). Compound-10 is the analogue of Compound-7, which strongly induced TOMM6 (cf. Fig. 7). The in vivo activity of Compound-10 and its fluorinated derivative, Compound-10F (Fig. 15A-C), was investigated. The synthesis scheme of Compound-10F, which was not synthesized before, is shown (Fig. 15D-G). Compound-10F was developed because the introduction of a fluorine into a small molecule can modulate various pharmacokinetic and physicochemical properties such as metabolic stability and enhanced membrane permeation (Shah and Westwell, J. Enzyme Inhibition Med. Chem. 22, 527-540, 2007; Böhm et al., Chembiochem 5, 637-643. 2004). Another potential application of the fluorine atom is the potential use of 18F as a radiolabel tracer atom in positron-emission tomography (PET) imaging (Shah and Westwell, J. Enzyme Inhibition Med. Chem. 22, 527-540, 2007).

### Example 16: Pharmacokinetic data of Compound-10F.

The pharmacokinetic parameters of Compound-10F were determined. Compound-10F had oral bioavailability in vivo, in rats and dogs (Fig. 16A-J). At t=1h after oral intake, the serum peak concentration of Compound-10F was achieved (Fig. 16B-I). The oral bioavailability of Compound-10F in dogs was much higher than in rats because oral doses of Compound-10F required to achieve equivalent peak serum concentrations were approximately 10-fold higher in rats compared to dogs (Fig. 16J). Again, this observation was in agreement with the equivalent surface area dosage rule, according to which rats and mice usually require a 10-12-fold higher dose than dogs and humans.

### Example 17: Compound-10F is a Tomm6 inducer and retards symptoms of AD and neurodegeneration in vivo.

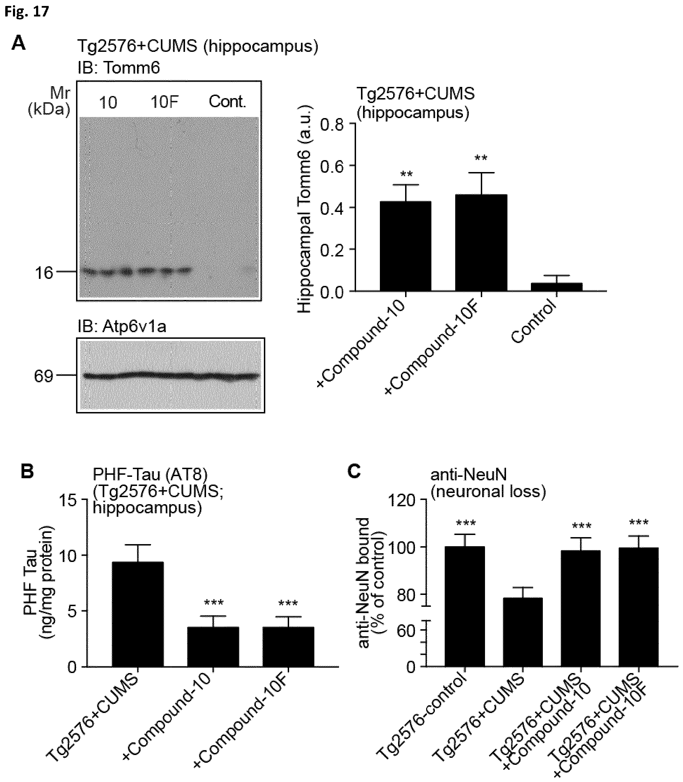

It was investigated whether Compound-10F is also a Tomm6 inducer in vivo. Hippocampal contents of Tomm6 were determined of 15-month-old Tg2576 AD mice subjected to the CUMS protocol for 3 months by immunoblot. Upon treatment for three months with Compound-10 and Compound-10F during the CUMS protocol at a daily oral dose of 10 mg/kg/d, the hippocampal Tomm6 contents of treated Tg2576 AD mice were significantly higher than those of untreated Tg2576 AD controls (Fig. 17A). Concomitantly, treatment with Compound-10 and Compound-10F significantly inhibited the hippocampal accumulation of hyperphosphorylated PHF tau and retarded the CUMS-induced hippocampal neuronal loss in Tg2576 AD mice (Fig. 17B, C). During 3 months of treatment at a daily dose of 10 mg/kg, Compound-10 and Compound-10F did not show any detectable toxic adverse effects. In view of these in vivo data, the present invention in general and for all embodiments encompasses the medical use in general and as described herein of Compound-10F, which was not synthesized before. Preferably, the present invention in general and for all embodiments encompasses the medical use of Compound-10F as Tomm6/TOMM6 inducer for the treatment and prophylaxis of nervous system diseases, neurodegenerative diseases, atherosclerosis and atherosclerosis-related diseases. The present invention in general and for all embodiments further encompasses the medical use, preferably as described herein, of (A) halogenated Compound-10, and Compound-7 and (B) of esters, ethers and amides of Compound-7 and Compound-10. Furthermore, the present invention in general and for all embodiments encompasses the medical use, preferably as described herein, of Compound-7, Compound-10 and Compound-10F, in which the 2-methyl group is removed or replaced by other substituents, e.g. by a halogene substituent.

### Example 18: Compound-10F prevents hippocampal PHF tau hyperphosphorylation and peripheral Agtr2 aggregation in the CUMS model with early symptoms of sporadic AD.

The Tg2576 AD mouse model recapitulates major features of familial AD (FAD), which only encompasses 1-3 % of all AD cases. The most frequent form of AD is the sporadic form of AD, which usually is not caused by a single disease-causing mutation (Quitterer and AbdAlla, Pharmacol Res. 2019 Apr 13. pii: S1043-6618(18)31950-9). To recapitulate features of early sporadic AD, the CUMS model was used and aged, 15-month-old rats were subjected to 4 weeks of CUMS (AbdAlla et al., Biomed Res. Int. 2015:917156, 2015). Hippocampal contents of hyperphosphorylated PHF tau were strongly increased by 4 weeks of CUMS (Fig. 18A). Treatment with the representative Tomm6-inducing Compound-10F (10 mg/kg/d) during the CUMS protocol prevented the hippocampal accumulation of hyperphosphorylated PHF tau (Fig. 18A). Thus, Compound-10F prevents a major neuropathologic feature in the hippocampus of a model of early sporadic AD. It was then asked whether the neuroprotective CNS treatment effect can be monitored in peripheral blood mononuclear cells (PBMC) with a peripheral blood cell marker. Tomm6/TOMM6 induction prevents the misfolding and aggregation of the neuroprotective angiotensin II type 2 receptor (AT2R) encoded by Agtr2/AGTR2 (cf. Fig. 3). To investigate the usefulness of Agtr2/AGTR2 as a peripheral blood cell marker for treatment outcome, peripheral blood mononuclear cells (PBMCs) were isolated from the CUMS rat model of sporadic AD. Immunoblot detection of Agtr2 showed that four weeks of CUMS had induced the aggregation and misfolding of Agtr2 in PBMCs (Fig. 18B). Treatment with Compound-10F, which prevented hippocampal PHF tau hyperphosphorylation (cf. Fig. 18A), also prevented the accumulation of aggregated Agtr2 in PBMCs of aged rats subjected to CUMS (Fig. 18B). The PBMC content of monomeric and neuroprotective Agtr2 was significantly higher compared to untreated controls (Fig. 18B). The PBMC content of aggregated Agtr2 protein was determined next by ELISA (Fig. 18C). The ELISA results showed that treatment with Compound-10F significantly retarded the CUMS-induced accumulation of Agtr2 aggregates in PBMC of aged rats (Fig. 18C). The increased content of aggregated Agtr2 in PBMC of CUMS rats as a model of sporadic AD is also related to AD pathology in human AD patients because the ELISA technique also detected an increased content of aggregated AGTR2 in PBMC of human patients with Alzheimer Disease compared to age-matched healthy controls without dementia (Fig. 18D). Taken together, CNS neuroprotective treatment effects of the Tomm6/TOMM6-inducing Compound-10F as a prototypic TOMM6/Tomm6 inducer can be monitored in peripheral blood cells by detection of Agtr2/AGTR2 and quantitation of monomeric and aggregated Agtr2/AGTR2. In view of the above, the present invention in general and for all embodiments encompasses the detection of monomeric, dimeric, oligomeric and misfolded/aggregated AGTR2/Agtr2 in peripheral blood (mononuclear) cells as a peripheral marker to monitor the neuroprotective treatment effect of TOMM6/Tomm6 inducers. In addition, the present invention in general and for all embodiments encompasses the use of the detection of Agtr2/AGTR2 as a peripheral blood marker to monitor neurodegeneration and treatment outcome with neuroprotective compounds.

## Claims

1. A TOMM6 (Translocase of Outer Membrane 6kDa subunit homolog)-interacting compound according to Formula (II) 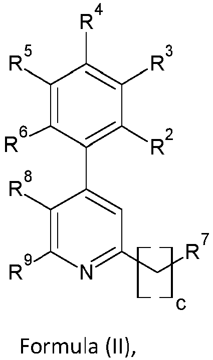 wherein
**R²** to **R⁶** are each independently selected from the group consisting of
(i) H, Cl, Br, F, -CN, or -OH,
(ii) -O(C₁₋₁₀)alkyl, preferably -OMe and -OEt, or -O(C₃₋₁₀)cycloalkyl,
(iii) -O(C=O)(C₁₋₁₀)alkyl, preferably -O(C=O)Me, or -O(C=O)(C₃₋₁₀)cycloalkyl,
(iv) -COOH, -COO(C₁₋₁₀)alkyl, -COO(C₃₋₆)cycloalkyl, -COONH₂, -COON((C₁₋₁₀)alkyl)₂, or - COONH(C₁₋₁₀)alkyl, preferably -COOH, -COOMe or COOEt,
preferably R⁴ is Cl and R², R³, R⁵ and R⁶ are H, more preferably R² and R⁴ are Cl and R³, R⁵ and R⁶ are H;
**R⁷** is selected from the group consisting of
(i) NH₂, NH(C₁₋₁₀)alkyl, -OH, -O(C₁₋₁₀)alkyl, or -CN, preferably -NH₂ or -OH;
(ii) -COOH, -COO(C₁₋₁₀)alkyl, -COO(C₃₋₆)cycloalkyl, -COONH₂, -COON((C₁₋₁₀)alkyl)₂, or - COONH(C₁₋₁₀)alkyl, preferably -COOH, -COOMe or COOEt;
(iii) substituted or non-substituted carbocycle selected from the group consisting of (C₃₋₁₀)carbocycle, preferably (C₃)carbocycle and (C₅₋₆)carbocycle, preferably aromatic (C₆)carbocycle, more preferably a non-substituted phenyl or a phenyl that is substituted by a substituent selected from the group consisting of Cl, F, Br, substituted or non-substituted methyl, preferably -(CF₃), ethyl, propyl and cyclopropyl; and
(iv) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 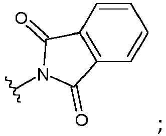
**R⁸** is selected from the group consisting of -C(=O)NH₂, -CN (instead of R⁸-CO), -C(=O)NH(C₁₋₁₀)alkyl, -C(=O)OH, or -C(=O)O(C₁₋₁₀)alkyl, preferably -C(=O)NH₂ or -CN;
**R⁹** is selected from the group consisting of
(i) -H, -Me, -Et, halogen, preferably -F, or -CN;
(ii) -COOH, -COO(C₁₋₁₀)alkyl, -COO(C₃₋₆)cycloalkyl, -COONH₂, -COON((C₁₋₁₀)alkyl)₂, or - COONH(C₁₋₁₀)alkyl, preferably -COOH, -COOMe or COOEt;
(iii) -OH, -O(C₁₋₁₀)alkyl, preferably -OMe and -OEt, or -O(C₃₋₁₀)cycloalkyl;
(iv) substituted or non-substituted (C₃₋₁₀)carbocycle, preferably (C₃)carbocycle and (C₅₋₆)carbocycle, preferably aromatic (C₆)carbocycle, more preferably non-substituted phenyl or phenyl substituted by a substituent selected from the group consisting of Cl, F, Br, substituted or non-substituted methyl, preferably -(CF₃), ethyl, propyl and cyclopropyl; and
**c** is an integer between 0 and 4;
and pharmaceutically acceptable salts and solvates thereof;
for use in the treatment or prophylaxis of a disease selected from the group consisting of:
(a) a nervous system disease or disorder, preferably a human nervous system disease or disorder, preferably a central nervous system (CNS) or peripheral nervous system (PNS) disease or disorder;
(b) atherosclerosis;
(c) Hepatitis B infection; and
(d) human papilloma virus (HPV) infection.

2. The compound according to Formula (II) for use according to claim 1, wherein
**R²** to **R⁶** are each independently selected from the group consisting of H, Cl, Br, or F, preferably R⁴ is Cl or F and R², R³, R⁵ and R⁶ are H or F, more preferably R² and R⁴ are Cl or F and R³, R⁵ and R⁶ are H or F;
**R⁷** is selected from the group consisting of
(i) -NH₂, -NH(C₁₋₁₀)alkyl, -OH, -CN, preferably -NH₂ or -OH;
(ii) -O(C₁₋₁₀)alkyl, preferably -OMe and -OEt, or -O(C₃₋₁₀)cycloalkyl;
(iii) -COOH, -COO(C₁₋₁₀)alkyl, -COO(C₃₋₆)cycloalkyl, -COONH₂, -COON((C₁₋₁₀)alkyl)₂, or - COONH(C₁₋₁₀)alkyl, preferably -COOH, -COOMe or COOEt; and
(iv) substituted or non-substituted (C₃₋₆)heterocycle or (C₇₋₁₀)carbo- or hetero-bicycle having 1 to 3 heteroatoms each independently selected from N, O and S, preferably substituted or non-substituted heterobicycle having 1 nitrogen atom, more preferably isoindoline-1,3-dione, most preferably 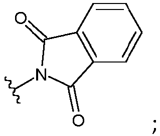
**R⁸** is -C(=O)NH₂ or -CN;
**R⁹** is selected from the group consisting of
(i) -H, -Me, -Et, halogen, preferably F, or -CN;
(ii) -COOH, -COO(C₁₋₁₀)alkyl, -COO(C₃₋₆)cycloalkyl, -COONH₂, -COON((C₁₋₁₀)alkyl)₂, or - COONH(C₁₋₁₀)alkyl, preferably -COOH, -COOMe or COOEt;
(iii) -OH, -O(C₁₋₁₀)alkyl, preferably -OMe and -OEt, or -O(C₃₋₁₀)cycloalkyl; and
(iv) substituted or non-substituted (C₃₋₁₀)carbocycle, preferably (C₃)carbocycle and (C₅₋₆)carbocycle, preferably aromatic (C₆)carbocycle, more preferably non-substituted phenyl or phenyl substituted by a substituent selected from the group consisting of Cl, F, Br, substituted or non-substituted methyl, preferably -(CF₃), ethyl, propyl and cyclopropyl; and/or
**c** is 0 or 1.

3. The compound according to Formula (II) for use according to claim 1 or 2, wherein the compound is selected from the group consisting of 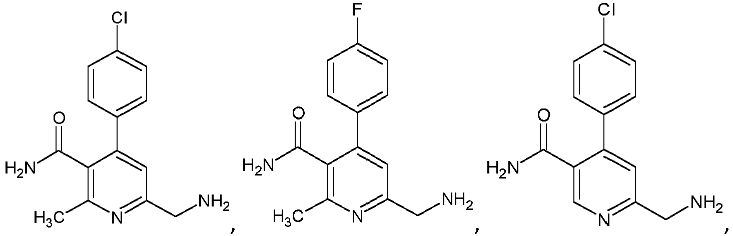 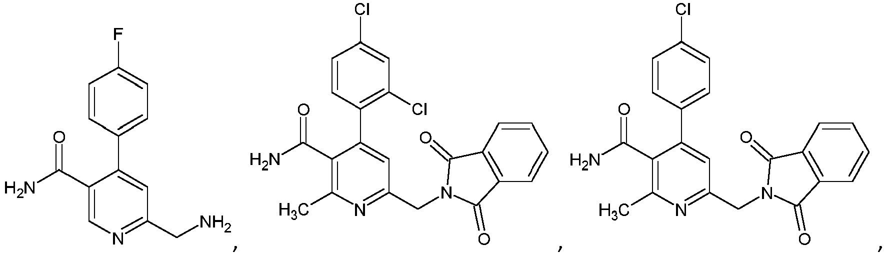 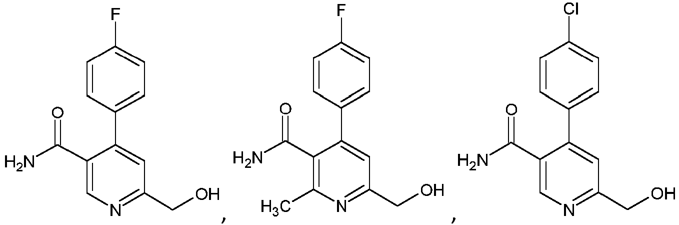 and

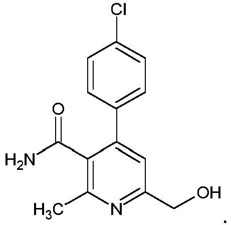

4. The TOMM6 (Translocase of Outer Membrane 6kDa subunit homolog)-interacting compound for use according to any of claims 1 to 3, wherein the nervous system disease or disorder is selected from the group consisting of:
brain injuries; cerebrovascular diseases; consequences of cerebrovascular diseases; motor neuron disease; dementias; ALS; multiple sclerosis; traumatic brain injury; small-vessel cerebrovascular disease; familial forms of Alzheimer's Disease; sporadic forms of Alzheimer's Disease; vascular dementia; subcortical leukoencephalopathy and subcortical atherosclerotic encephalopathy; mixed forms of dementia; M. Parkinson; progressive supranuclear palsy and other forms of atypical parkinsonism; frontotemporal dementia; subcortical dementia;
CADASIL (cerebral autosomal dominant arteriopathy with subcortical infarcts and
leukoencephalopathy); cerebral palsy; encephalitis lethargica; corticobasal degeneration;
multiple system atrophy; chronic traumatic encephalopathy; Lytico-Bodig disease; FTDP-17;
Parkinson linked to chromosome-17; diabetic neuropathy; symptoms of depression and
depression-related symptoms, preferably anhedonia and anorexia; schizophrenia with dementia; Korsakoff's psychosis; Lewy Body diseases; progressive supranuclear palsy;
corticobasal degeneration; Pick's disease; Huntington's disease; thalamic degeneration;
Creutzfeld-Jacob disease; HIV Dementia; disorders with mitochondrial dysfunction, preferably neurodegenerative diseases of ageing; cognitive-related disorder; mild cognitive impairment;
age-associated memory impairment; age-associated cognitive decline; vascular cognitive impairment; central and peripheral symptoms of atherosclerosis and ischemia;
atherosclerosis-related cardiovascular diseases; stroke; perivascular disease; renal dysfunction and renal failure; stress-related disorders; attention deficit disorders; attention deficit hyperactivity disorders; and memory disturbances in children.

## Patentansprüche

1. Eine mit TOMM6 (Translokase der Aussenmembran 6kDa homologen Untereinheit)-wechselwirkende Verbindung der Formel (II) 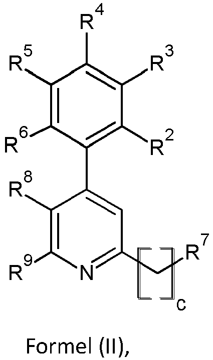 wobei
**R²** bis **R⁶** jeweils unabhängig voneinander aus der Gruppe ausgewählt sind, bestehend aus
(i) H, Cl, Br, F, -CN, oder -OH,
(ii) -O(C₁₋₁₀)Alkyl, vorzugsweise -OMe und -OEt, oder -O(C₃₋₁₀)Cycloalkyl,
(iii) -O(C=O)(C₁₋₁₀)Alkyl, vorzugsweise -O(C=O)Me, oder -O(C=O)(C₃₋₁₀)Cycloalkyl,
(iv) -COOH, -COO(C₁₋₁₀)Alkyl, -COO(C₃₋₆)Cycloalkyl, -COONH₂, -COON((C₁₋₁₀)Alkyl)₂, oder -COONH(C₁₋₁₀)Alkyl, vorzugsweise -COOH, -COOMe oder COOEt,
vorzugsweise R⁴ Cl ist und R², R³, R⁵ und R⁶ H sind, mehr bevohrzugt R² und R⁴ Cl sind und R³, R⁵ und R⁶ H sind;
**R⁷** aus der Gruppe ausgewählt ist, bestehend aus
(i) NH₂, NH(C₁₋₁₀)Alkyl, -OH, -O(C₁₋₁₀)Alkyl, oder -CN, vorzugsweise -NH₂ oder -OH;
(ii) -COOH, -COO(C₁₋₁₀)Alkyl, -COO(C₃₋₆)Cycloalkyl, -COONH₂, -COON((C₁₋₁₀)Alkyl)₂, oder -COONH(C₁₋₁₀)Alkyl, vorzugsweise -COOH, -COOMe oder COOEt;
(iii) substituiertem oder nicht-substituiertem Carbocyclus, ausgewählt aus der Gruppe bestehend aus (C₃₋₁₀)Carbocyclus, vorzugsweise (C₃)Carbocyclus und (C₅₋₆)Carbocyclus, vorzugsweise aromatischem (C₆)Carbocyclus, mehr bevorzugt ein nicht-substituiertes Phenyl oder ein Phenyl, das mit einem Substituenten substituiert ist, der aus der Gruppe ausgewählt ist, bestehend aus Cl, F, Br, substituiertem oder nicht-substituieretem Methyl, vorzugsweise -(CF₃), Ethyl, Propyl und Cyclopropyl; und
(iv) substituiertem oder nicht-substituiertem (C₃₋₆)Heterocyclus oder (C₇₋₁₀)Carbo- oder Heterobicyclus mit 1 bis 3 Heteroatomen, jedes davon unabhängig voneinander ausgewählt aus N, O und S, vorzugsweise substituierter oder nicht-substituierter Heterobicyclus mit 1 Stickstoffatom, mehr bevorzugt Isoindolin-1,3-dion, mehr bevorzugt 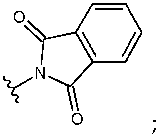
**R⁸** aus der Gruppe ausgewählt ist, bestehend aus -C(=O)NH₂, -CN (anstatt R⁸-CO), -C(=O)NH(C₁₋₁₀)Alkyl, -C(=O)OH, oder -C(=O)O(C₁₋₁₀)Alkyl, vorzugsweise -C(=O)NH₂ oder -CN;
**R⁹** aus der Gruppe ausgewählt ist, bestehend aus
(i) -H, -Me, -Et, Halogen, vorzugsweise -F oder -CN;
(ii) -COOH, -COO(C₁₋₁₀)Alkyl, -COO(C₃₋₆)Cycloalkyl, -COONH₂, -COON((C₁₋₁₀)Alkyl)₂, oder -COONH(C₁₋₁₀)Alkyl, vorzugsweise -COOH, -COOMe oder COOEt;
(iii) -OH, -O(C₁₋₁₀)Alkyl, vorzugsweise -OMe und -OEt, oder -O(C₃₋₁₀)Cycloalkyl;
(iv) substituiertem oder nicht-substituiertem (C₃₋₁₀)Carbocyclus, vorzugsweise (C₃)Carbocyclus und (C₅₋₆)Carbocyclus, vorzugsweise aromatischer (C₆)Carbocyclus, mehr bevorzugt nicht-substituiertes Phenyl oder Phenyl, das mit einem Substituenten substituiert ist, ausgewählt aus der Gruppe bestehend aus Cl, F, Br, substituiertem oder nicht-substituiertem Methyl, vorzugsweise -(CF₃), Ethyl, Propyl und Cyclopropyl; und
**c** eine ganze Zahl zwieschen 0 und 4 ist;
und pharmazeutisch verträgliche Salze und Solvate davon;
zur Verwendnug in der Behandlung oder Prophylaxe einer Erkrankung, ausgewählt aus der Gruppe bestehend aus:
(a) einer Erkrankung oder Störung des Nervensystems, vorzugsweise einer Erkrankung oder Störung des menschlichen Nervensystems, vorzugsweise einer Erkrankung oder Störung des zentralen Nervensystems (ZNS) oder des peripheren Nervensystems (PNS);
(b) Atherosklerose;
(c) Hepatitis-B-Infektion; und
(d) Infektion mit dem menschlichen Papillomavirus (HPV).

2. Die Verbindung der Forme (II) zur Verwendung nach Anspruch 1, wobei
**R²** bis **R⁶** unabhängig voneinander aus der Gruppe ausgewählt sind, bestehend aus H, Cl, Br, oder F,
vorzugsweise R⁴ Cl oder F ist und R², R³, R⁵ und R⁶ H oder F sind, mehr bevorzugt R² und R⁴ Cl oder F sind und R³, R⁵ und R⁶ H oder F sind;
**R⁷** aus der G>ruppe ausgewählt ist, bestehend aus:
(i) -NH₂, -NH(C₁₋₁₀)Alkyl, -OH, -CN, vorzugsweise -NH₂ oder -OH;
(ii) -O(C₁₋₁₀)Alkyl, vorzugsweise -OMe und -OEt, oder -O(C₃₋₁₀)Cycloalkyl;
(iii) -COOH, -COO(C₁₋₁₀)Alkyl, -COO(C₃₋₆)Cycloalkyl, -COONH₂, -COON((C₁₋₁₀)Alkyl)₂, oder -COONH(C₁₋₁₀)Alkyl, vorzugsweise -COOH, -COOMe oder COOEt; und
(iv) substituiertem oder nicht-substituiertem (C₃₋₆)Heterocyclus oder (C₇₋₁₀)Carbo- oder Hetero-bicyclus mit 1 bis 3 Heteroatomen, wobei jedes davon unabhängig voneinander ausgewählt ist aus N, O und S, vorzugsweise substituierter oder nicht-substituierter Heterobicyclus mit 1 Stickstoffatom, mehr bevorzuugt Isoindolin-1,3-dion, am meisten bevorzugt 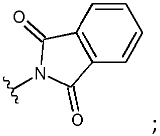
**R⁸** -C(=O)NH₂ oder -CN ist;
**R⁹** aus der Gruppe ausgewählt ist, bestehend aus
(i) -H, -Me, -Et, Halogen, vorzugsweise F oder -CN;
(ii) -COOH, -COO(C₁₋₁₀)Alkyl, -COO(C₃₋₆)Cycloalkyl, -COONH₂, -COON((C₁₋₁₀)Alkyl)₂, oder -COONH(C₁₋₁₀)Alkyl, vorzugsweise -COOH, -COOMe oder COOEt;
(iii) -OH, -O(C₁₋₁₀)Alkyl, vorzusweise -OMe und -OEt, oder -O(C₃₋₁₀)Cycloalkyl; und
(iv) substituiertem oder nicht-substituiertem (C₃₋₁₀)Carbocyclus, vorzugsweise (C₃)Carbocyclus und (C₅₋₆)Carbocyclus, vorzugsweise aromatischer (C₆)Carbocyclus, mehr bevorzugt nicht substiuertes Phenyl oder Phenyl, das mit einem Substituenten substituiert ist, der aus der Gruppe ausgewählt ist, bestehend aus Cl, F, Br, substituiertem oder nicht-substituiertem Methyl, vorzugsweise -(CF₃), Ethyl, Propyl und Cyclopropyl; und/oder
**c** 0 oder 1 ist.

3. Die Verbindung der Formel (II) zur Verwendung nach Anspruch 1 oder 2, wobei die Verbindung aus der Gruppe ausgewählt ist, bestehend aus 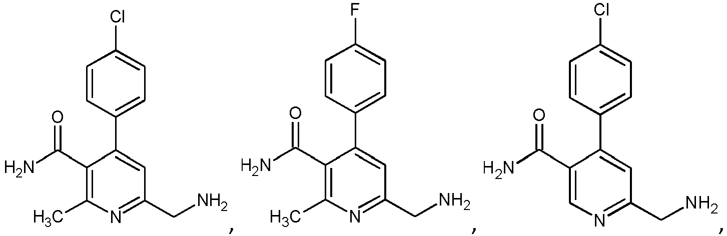 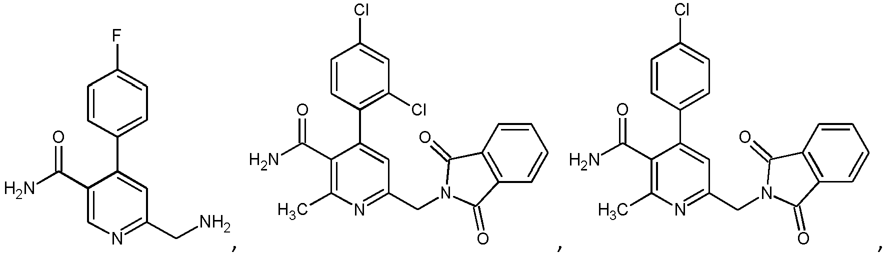 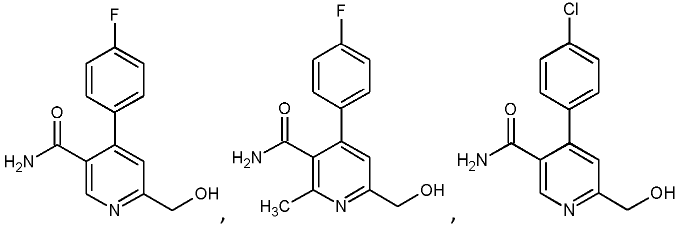 und

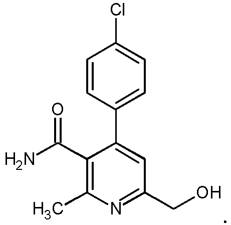

4. Die mit TOMM6 (Translokase der Aussenmembran 6kDa homologen Untereinheit)-wechselwirkende Verbindung zur Verwendung nach einem der Ansprüche 1 bis 3, wobei die Erkrankung oder Störung des Nervensystems aus der Gruppe ausgewählt ist, bestehend aus:
Hirnverletzungen; zerebrovaskuläre Erkrankungen; Folgen zerebrovaskulärer Erkrankungen;
Motoneuronenerkrankung; Demenzen; ALS; Multiple Sklerose; traumatische Hirnverletzung;
kleingefäßige zerebrovaskuläre Erkrankung; familiäre Formen der Alzheimer-Erkrankung;
sporadische Formen der Alzheimer- Erkrankung; vaskuläre Demenz; subkortikale Leukoenzephalopathie und subkortikale atherosklerotische Enzephalopathie; gemischte Formen der Demenz; M. Parkinson; progressive supranukleäre Lähmung und andere Formen des atypischen Parkinsonismus; frontotemporale Demenz; subkortikale Demenz; CADASIL (zerebrale autosomal dominante Arteriopathie mit subkortikalen Infarkten und Leukoenzephalopathie);
Zerebralparese; Enzephalitis lethargica; kortikobasale Degeneration; multiple Systematrophie; chronische traumatische Enzephalopathie; Lytico-Bodig-Erkrankung; FTDP-17; Parkinson in Verbindung mit Chromosom-17; diabetische Neuropathie; Symptome einer Depression und mit Depression-assiziierte Symptome, vorzugsweise Anhedonie und Anorexie; Schizophrenie mit Demenz; Korsakoff's-Psychose; Lewy-Body-Erkrankungen; progressive supranukleäre Lähmung; kortikobasale Degeneration; Pick-Erkrankung; Huntington's-Erkrankung; Thalamusdegeneration; Creutzfeld-Jacob-Erkrankung; HIV-Demenz; Erkrankungen mit mitochondrialer Dysfunktion, vorzugsweise neurodegenerative Erkrankungen des Alterns; kognitive Störungen; leichte kognitive Beeinträchtigung; alters-assoziierte Gedächtnisstörung; alters-assoziierter kognitiver Abbau; vaskuläre kognitive Beeinträchtigung; zentrale und periphere Symptome von Atherosklerose und Ischämie; atherosklerose-assoziierte kardiovaskuläre Erkrankungen; Schlaganfall; perivaskuläre Erkrankung; Nierenfunktionsstörung und
Nierenversagen; stress-assoziierte Störungen; Aufmerksamkeitsdefizitstörungen;
Aufmerksamkeitsdefizit-Hyperaktivitätsstörungen; und Gedächtnisstörungen bei Kindern.

## Revendications

1. Composé interagissant avec TOMM6 (translocase de l'homologue de sous-unité de 6 kDa de la membrane externe) selon la Formule (II) : 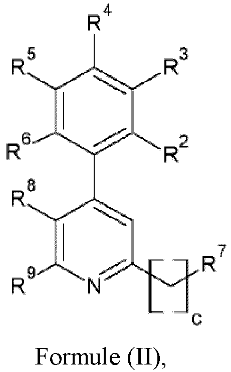 où :
R² à R⁶ sont chacun indépendamment choisis dans le groupe constitué de :
(i) H, Cl, Br, F, -CN ou -OH,
(ii) -O(C₁₋₁₀)alkyle, de préférence -OMe et -OEt, ou -O(C₃₋₁₀)cycloalkyle,
(iii) -O(C=O)(C₁₋₁₀)alkyle, de préférence -O(C=O)Me, ou -O(C=O)(C₃₋₁₀)cycloalkyle,
(iv) -COOH, -COO(C₁₋₁₀)alkyle, -COO(C₃₋₆)cycloalkyle, -COONH₂, -COON((C₁₋₁₀)alkyle)₂ ou -COONH(C₁₋₁₀)alkyle, de préférence -COOH, -COOMe ou -COOEt,
de préférence R⁴ est Cl et R², R³, R⁵ et R⁶ sont H, plus préférablement R² et R⁴ sont Cl et R³, R⁵ et R⁶ sont H ;
R⁷ est choisi dans le groupe constitué de :
(i) NH₂, NH(C₁₋₁₀)alkyle, -OH, -O(C₁₋₁₀)alkyle ou -CN, de préférence -NH₂ ou -OH ;
(ii) -COOH, -COO(C₁₋₁₀)alkyle, -COO(C₃₋₆)cycloalkyle, -COONH₂, -COON((C₁₋₁₀)alkyle)₂ ou -COONH(C₁₋₁₀)alkyle, de préférence -COOH, -COOMe ou -COOEt ;
(iii) carbocycle substitué ou non substitué choisi dans le groupe constitué de : (C₃₋₁₀)carbocycle, de préférence (C₃)carbocycle et (C₅₋₆)carbocycle, de préférence (C₆)carbocycle aromatique, plus préférablement un phényle non substitué ou un phényle qui est substitué par un substituant choisi dans le groupe constitué de : Cl, F, Br, méthyle substitué ou non substitué, de préférence -(CF₃), éthyle, propyle et cyclopropyle ; et
(iv) (C₃₋₆)hétérocycle ou (C₇₋₁₀)carbo- ou hétéro-bicycle substitués ou non substitués possédant 1 à 3 hétéroatomes, chacun indépendamment choisi parmi N, O et S, de préférence hétérobicycle substitué ou non substitué possédant 1 atome d'azote, plus préférablement isoindoline-1,3-dione, le plus préférablement 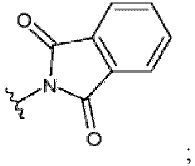
R⁸ est choisi dans le groupe constitué de : -C(=O)NH₂, -CN (à la place de R⁸-CO), -C(=O)NH(C₁₋₁₀)alkyle, -C(=O)OH ou -C(=O)O(C₁₋₁₀)alkyle, de préférence -C(=O)NH₂ ou -CN ;
R⁹ est choisi dans le groupe constitué de :
(i) -H, -Me, -Et, halogène, de préférence -F, ou -CN ;
(ii) -COOH, -COO(C₁₋₁₀)alkyle, -COO(C₃₋₆)cycloalkyle, -COONH₂, -COON((C₁₋₁₀)alkyle)₂ ou -COONH(C₁₋₁₀)alkyle, de préférence -COOH, -COOMe ou -COOEt ;
(iii) -OH, -O(C₁₋₁₀)alkyle, de préférence -OMe et -OEt, ou -O(C₃₋₁₀)cycloalkyle ;
(iv) (C₃₋₁₀)carbocycle substitué ou non substitué, de préférence (C₃)carbocycle et (C₅₋₆)carbocycle, de préférence (C₆)carbocycle aromatique, plus préférablement un phényle non substitué ou un phényle substitué par un substituant choisi dans le groupe constitué de : Cl, F, Br, méthyle substitué ou non substitué, de préférence -(CF₃), éthyle, propyle et cyclopropyle ; et
c est un nombre entier entre 0 et 4 ;
et des sels et solvates pharmaceutiquement acceptables de celui-ci ;
pour utilisation dans le traitement ou la prophylaxie d'une maladie choisie dans le groupe constitué de :
(a) une maladie ou un trouble du système nerveux, de préférence une maladie ou un trouble du système nerveux humain, de préférence une maladie ou un trouble du système nerveux central (SNC) ou du système nerveux périphérique (SNP) ;
(b) une athérosclérose ;
(c) une infection par l'hépatite B ; et
(d) une infection par le papillomavirus humain (PVH).

2. Composé selon la Formule (II) pour utilisation selon la revendication 1, dans lequel :
R² à R⁶ sont chacun indépendamment choisis dans le groupe constitué de : H, Cl, Br ou F,
de préférence R⁴ est Cl ou F et R², R³, R⁵ et R⁶ sont H ou F, plus préférablement R² et R⁴ sont Cl ou F et R³, R⁵ et R⁶ sont H ou F ;
R⁷ est choisi dans le groupe constitué de :
(i) NH₂, NH(C₁₋₁₀)alkyle, -OH, -CN, de préférence -NH₂ ou -OH ;
(ii) -O(C₁₋₁₀)alkyle, de préférence -OMe et -OEt, ou -O(C₃₋₁₀)cycloalkyle ;
(iii) -COOH, -COO(C₁₋₁₀)alkyle, -COO(C₃₋₆)cycloalkyle, -COONH₂, -COON((C₁₋₁₀)alkyle)₂ ou -COONH(C₁₋₁₀)alkyle, de préférence -COOH, -COOMe ou -COOEt ; et
(iv) (C₃₋₆)hétérocycle ou (C₇₋₁₀)carbo- ou hétéro-bicycle substitués ou non substitués possédant 1 à 3 hétéroatomes, chacun indépendamment choisi parmi N, O et S, de préférence hétérobicycle substitué ou non substitué possédant 1 atome d'azote, plus préférablement isoindoline-1,3-dione, le plus préférablement 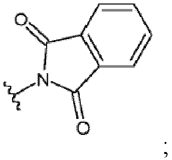
R⁸ est -C(=O)NH₂ ou -CN ;
R⁹ est choisi dans le groupe constitué de :
(i) -H, -Me, -Et, halogène, de préférence -F, ou -CN ;
(ii) -COOH, -COO(C₁₋₁₀)alkyle, -COO(C₃₋₆)cycloalkyle, -COONH₂, -COON((C₁₋₁₀)alkyle)₂ ou -COONH(C₁₋₁₀)alkyle, de préférence -COOH, -COOMe ou -COOEt ;
(iii) -OH, -O(C₁₋₁₀)alkyle, de préférence -OMe et -OEt, ou -O(C₃₋₁₀)cycloalkyle ; et
(iv) (C₃₋₁₀)carbocycle substitué ou non substitué, de préférence (C₃)carbocycle et (C₅₋₆)carbocycle, de préférence (C₆)carbocycle aromatique, plus préférablement un phényle non substitué ou un phényle substitué par un substituant choisi dans le groupe constitué de : Cl, F, Br, méthyle substitué ou non substitué, de préférence -(CF₃), éthyle, propyle et cyclopropyle ; et/ou
c est 0 ou 1.

3. Composé selon la Formule (II) pour utilisation selon la revendication 1 ou 2, où le composé est choisi dans le groupe constitué de : 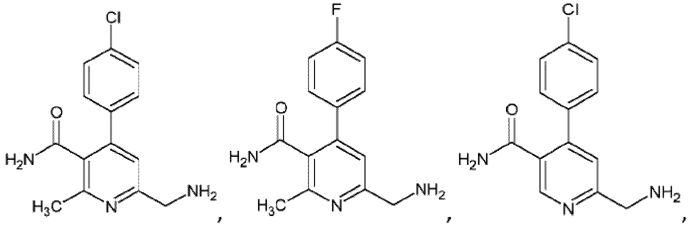 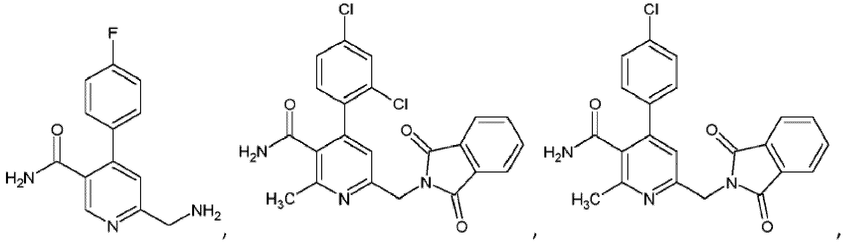 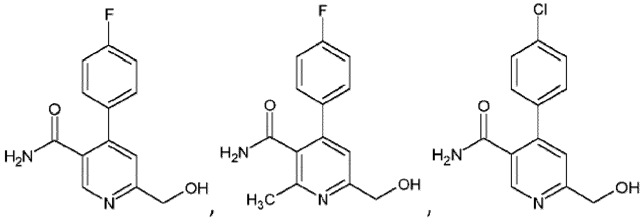 et

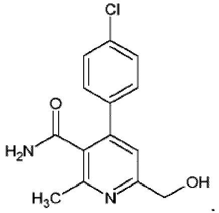

4. Composé interagissant avec TOMM6 (translocase de l'homologue de sous-unité de 6 kDa de la membrane externe) pour utilisation selon l'une quelconque des revendications 1 à 3, où la maladie ou le trouble du système nerveux est choisi dans le groupe constitué de :
lésions du cerveau ; maladies cérébrovasculaires ; conséquences de maladies cérébrovasculaires ; maladie du neurone moteur ; démences ; ALS ; sclérose en plaques ; lésion du cerveau traumatique ; maladie cérébrovasculaire sur les vaisseaux de petit calibre ; formes familiales de la maladie d'Alzheimer ; formes sporadiques de la maladie d'Alzheimer ; démence vasculaire ; leucoencéphalopathie sous-corticale et encéphalopathie athérosclérotique sous-corticale ; formes mixtes de démence ; maladie de Parkinson ; paralysie supranucléaire progressive et autres formes de parkinsonisme atypique ; démence fronto-temporale ; démence sous-corticale ; CADASIL (artériopathie dominante autosomale cérébrale avec infarctus et leucoencéphalopathie sous-corticaux) ; paralysie cérébrale ; encéphalite léthargique ; dégénérescence corticobasale ; atrophie multisystématisée ; encéphalopathie traumatique chronique ; maladie de Lytico-Bodig ; FTDP-17 ; Parkinson lié au chromosome 17 ; neuropathie diabétique ; symptômes de dépression et symptômes liés à une dépression, de préférence anhédonie et anorexie ; schizophrénie avec démence ; psychose de Korsakoff ; maladies à corps de Lewy ; paralysie supranucléaire progressive ; dégénérescence corticobasale ; maladie de Pick ; maladie de Huntington ; dégénérescence thalamique ; maladie de Creutzfeld-Jacob ; démence due au VIH ; troubles avec dysfonctionnement mitochondrial, de préférence maladies neurodégénératives du vieillissement ; trouble lié au cognitif ; trouble cognitif léger ; dégradation de la mémoire associée à l'âge ; diminution cognitive associée à l'âge ; dégradation cognitive vasculaire ; symptômes central et périphérique d'athérosclérose et d'ischémie ; maladies cardiovasculaires liées à une athérosclérose ; accident vasculaire cérébral ; maladie périvasculaire ; dysfonctionnement rénal et insuffisance rénale ; troubles liés au stress ; troubles de déficit de l'attention ; troubles de déficit de l'attention avec hyperactivité ; et troubles de la mémoire chez l'enfant.
